# EUROPEAN PATENT APPLICATION

(11) **EP 1 911 850 A1**
(43) Date of publication of application: **16.04.2008**
(21) Application number: 06291569.9
(22) Date of filing: 09.10.2006
(51) Int. Cl.: C12P 21/00, C12P 19/26, C12P 19/18, C12P 11/00

(54) **Method of producing 6-thio-sialylated glycoproteins and glycoconjugates**

(71) Applicant: CENTRE NATIONAL DE LA RECHERCHE SCIENTIFIQUE (CNRS), 75016 Paris (FR); UNIVERSITE JOSEPH FOURIER - Grenoble 1, 38400 St. Martin d'Hères (FR)
(72) Inventor: Fort, Sébastien, 38410 Uriage (FR); Cottaz, Sylvain, 38320 Herbeys (FR); Birikaki, Lémonia, 38000 Grenoble (FR); Samain, Eric, 38610 Gieres (FR); Driguez, Hugues, 38000 Grenoble (FR)
(74) Representative: Warcoin, Jacques

(57) **Abstract**

The present invention relates to a method of preparing 6-thio-sialyl-glycosyl compounds, comprising the step consisting of reacting *in vitro* or *in vivo* 6-thio-sialic acid compounds with a CMP-sialic synthetase to form CMP-6-thio sialic acid compounds and with a sialyltransferase to transfer said CMP-6-thio sialic acid compounds as donor substrate to glycosyl compounds as acceptor subtrate leading to the enzymatic coupling of 6-thiosialic acid compounds onto glycosyl compounds. Glycosyl compounds are selected from glycoproteins, glycolipids and oligosaccharides, including monoclonal antibodies.

SEQUENCE LISTING

<110> CENTRE NATIONAL DE LA RECHERCHE SCIENTIFIQUE
      UNIVERSITE JOSEPH FOURIER - GRENOBLE I

<120> Method of producing 6-thio-sialylated glycoproteins and
      glycoconjugates

<130> D24447

<160> 6

<170> PatentIn version 3.3

<210> 1
<211> 876
<212> DNA
<213> Campylobacter jejuni

<220>
<223> bifunctional alpha-2,3/alpha 2,8-sialyltransferase or
      sialyltransferase II (cstII) - NCBI AX934425
      Campylobacter jejuni strain OH4384

<400> 1
   atgaaaaaag ttattattgc tggaaatgga ccaagtttaa aagaaattga ttattcaaga      60
   ctaccaaatg attttgatgt atttagatgt aatcaatttt attttgaaga taaatactat     120
   cttggtaaaa aatgcaaggc agtattttac aatcctattc ttttttttga acaatactac     180
   actttaaaac atttaatcca aaatcaagaa tatgagaccg aactaattat gtgttctaat     240
   tacaaccaag ctcatctaga aaatgaaaat tttgtaaaaa ctttttacga ttattttcct     300
   gatgctcatt tgggatatga ttttttcaaa caacttaaag attttaatgc ttattttaaa     360
   tttcacgaaa tttatttcaa tcaaagaatt acctcagggg tttatatgtg tgcagtagcc     420
   atagccctag gatacaaaga aatttatctt tcgggaattg atttttatca aaatgggtca     480
   tcttatgctt ttgatactaa acaaaaaaat cttttaaaat tggctcctaa ttttaaaaat     540
   gataattcac actatatcgg acatagtaaa aatacagata taaaagcttt agaatttcta     600
   gaaaaaactt acaaaataaa actatattgc ttatgtccta acagtctttt agcaaatttt     660
   atagaactag cgccaaattt aaattcaaat tttatcatac aagaaaaaaa taactacact     720
   aaagatatac tcataccttc tagtgaggct tatggaaaat tttcaaaaaa tattaatttt     780
   aaaaaaataa aaattaaaga aaatatttat tacaagttga taaaagatct attaagatta     840
   cctagtgata taaagcatta tttcaaagga aaataa                               876

<210> 2
<211> 876
<212> DNA
<213> Campylobacter jejuni

<220>
<223> alpha-2,3/alpha 2,8-sialyltransferase or
      sialyltransferase II (cstII) - NCBI AX934427
      Campylobacter jejuni serotype O:10

<400> 2
atgaaaaaag ttattattgc tggaaatgga ccaagtttaa aagaaattga ttattcaagg      60
ctaccaaatg attttgatgt atttagatgc aatcaatttt attttgaaga taaatactat     120
cttggtaaaa aattcaaagc agtattttac aatcctggtc ttttttttga acaatactac     180
actttaaaac atttaatcca aaatcaagaa tatgagaccg aactaattat gtgttctaat     240
tacaaccaag ctcatctaga aaatgaaaat tttgtaaaaa ctttttacga ttattttcct     300
gatgctcatt tgggatatga tttttttaaa caacttaaag aatttaatgc ttattttaaa     360
tttcacgaaa tttatctcaa tcaaagaatt acctcaggag tctatatgtg tgcagtagct     420
atagccctag gatacaaaga aatttatctt tctggaattg atttttatca aaatgggtca     480

tcttatgctt ttgataccaa acaagaaaat cttttaaaac tggctcctga ttttaaaaat 540
gatcgctcac actatatcgg acatagtaaa aatacagata taaaagcttt agaatttcta 600
gaaaaaactt acaaaataaa actatattgc ttatgtccta acagtctttt agcaaatttt 660
atagaactag cgccaaattt aaattcaaat tttatcatac aagaaaaaaa taactacact 720
aaagatatac tcataccttc tagtgaggct tatggaaaat tttcaaaaaa tattaatttt 780
aaaaaaataa aaattaaaga aaatatttat tacaagttga taaaagatct attaagatta 840
cctagtgata taaagcatta tttcaaagga aaataa 876

<210> 3
<211> 876
<212> DNA
<213> Campylobacter jejuni

<220>
<223> alpha-2,3/alpha 2,8-sialyltransferase II (cstII) - AX934429
      Campylobacter jejuni serotype O:41

<400> 3
atgaaaaaag ttattattgc tggaaatgga ccaagtttaa aagaaattga ttattcaaga 60
ctaccaaatg attttgatgt atttagatgc aatcaatttt attttgaaga taaatactat 120
cttggtaaaa aatgcaaagc agtattttac aatcctagtc ttttttttga acaatactac 180
actttaaaac atttaatcca aaatcaagaa tatgagaccg aactaatcat gtgttctaat 240
tttaaccaag ctcatctaga aaatcaaaat tttgtaaaaa ctttttacga ttattttcct 300
gatgctcatt tgggatatga ttttttcaaa caacttaaag aattcaatgc ttattttaaa 360
tttcacgaaa tttatttcaa tcaaagaatt acctcagggg tctatatgtg cacagtagcc 420
atagccctag gatacaaaga aatttatctt tcgggaattg atttttatca aaatggatca 480
tcttatgctt ttgataccaa acaaaaaaat cttttaaaat tggctcctaa ttttaaaaat 540
gataattcac actatatcgg acatagtaaa aatacagata taaaagcttt agaatttcta 600
gaaaaaactt acgaaataaa gctatattgt ttatgtccta acagtctttt agcaaatttt 660
atagaactag cgccaaattt aaattcaaat tttatcatac aagaaaaaaa taactatact 720
aaagatatac tcataccttc tagtgaggct tatggaaaat ttacaaaaaa tattaatttt 780
aaaaaaataa aaattaaaga aaatatttat tacaagttga taaaagatct attaagatta 840
cctagtgata taaagcatta tttcaaagga aaataa 876

<210> 4
<211> 2039
<212> DNA
<213> Neisseria meningitidis

<220>
<223> alpha-2,3-sialyltransferase gene - NCBI U60660

<400> 4
   ccggagagag aaatgtcgct ggttttgaca tcaatatcgg cgtggcgggc aaaagcctgc 60
   acaatcggca gcagcgattg ggtcgccagc gcgggtgctt cgtcggtatg ggtataaaca 120
   atggtggatt tttgagtcat aggattattc tcttgtaggt tggttttttc ttttggaaca 180
   cattgcgcgg ggaatgtgcg tggctattat ggcatatttt ggcggctttg ttcgcgcttt 240
   gttcgatctt ggcgtgtttg aacgcggcgg cgtgaaagga agggggaaat ggttttcccg 300
   cgtttggcgg cggtcggagg tgctgtgcct gatgtgcggc ggcatatttt cggtgaaatt 360
   gattttatag tggtttaaat ttaaaccagt acagcgttgc ctcgccttgt cgtactgctt 420
   gtactgtctg cggcttcgtc gccttgtcct gatttaaatt taaaccacta taatattcgg 480
   taactgtcgg aatatctgct aaaattccgc atttttccgt cccgggacac tcggggcgta 540
   tgttcaattt gtcggaatgg agttttaggg atatgggctt gaaaaaggct tgtttgaccg 600
   tgttgtgttt gattgttttt tgtttcggga tattttatac atttgaccgg gtaaatcagg 660
   gggaaaggaa tgcggtttcc ctgctgaagg agaaactttt caatgaagag ggggaaccgg 720
   tcaatctgat tttctgttat accatattgc agatgaaggt ggcggaaagg attatggcgc 780
   agcatccggg cgagcggttt tatgtggtgc tgatgtctga aaacaggaat gaaaaatacg 840
   attattattt caatcagata aaggataagg cggagcgggc gtactttttc cacctgccct 900
   acggtttgaa caaatcgttt aatttcattc cgacgatggc ggagctgaag gtaaagtcga 960
   tgctgctgcc gaaagtcaag cggatttatt tggcaagttt ggaaaaagtc agcattgccg 1020
   cctttttgag cacttacccg gatgcggaaa tcaaaacctt tgacgacggg acaggcaatt 1080
   taattcaaag cagcagctat ttgggcgatg agttttctgt aaacgggacg atcaagcgga 1140
   attttgcccg gatgatgatc ggagattgga gcatcgccaa aacccgcaat gcttccgacg 1200
   agcattacac gatattcaag ggtttgaaaa acattatgga cgacggccgc cgcaagatga 1260
   cttacctgcc gctgttcgat gcgtccgaac tgaagacggg ggacgaaacg ggcggcacgg 1320
   tgcggatact tttgggttcg cccgacaaag agatgaagga aatttcggaa aaggcggcaa 1380
   aaaacttcaa aatacaatat gtcgcgccgc atccccgcca aacctacggg ctttccggcg 1440
   taaccacatt aaattcgccc tatgtcatcg aagactatat tttgcgcgag attaagaaaa 1500
   acccgcatac gaggtatgaa atttatacct ttttcagcgg cgcggcgttg acgatgaagg 1560
   attttcccaa tgtgcacgtt tacgcattga aaccggcttc ccttccggaa gattattggc 1620
   tcaagccggt gtatgccctg tttacccaat ccggcatccc gattttgaca tttgacgata 1680
   aaaattaatc gcatagcaaa tcaaaataga aaatggcgga gtgcgtgggg taaaaataag 1740
   gatagcgttt tttcatttgg attgacgata atttctgatt gctttgcgtg tgctgaaatg 1800
   gcaaagaaaa tgccgtctga agtcttcaga cggcattgtt ttgttttgga tgttattcgg 1860
   gcgcgcggaa actgtcgtgg caggatttgc agcttgcgcc ggtttcgccg taggcggctt 1920
   tgatttcgtc cagtttgccg gtttggcggg cggcgttgag tttttcgacg gcggcggcga 1980
   attttgtttt ttcggcttca aattttgcac catccgacca aacggcgggc agtgcgcgg 2039

<210> 5
<211> 496
<212> PRT
<213> Escherichia coli

<220>
<223> sialic acid transporter - NCBI NP_417691
      Escherichia coli K12

<400> 5

Met Ser Thr Thr Thr Gln Asn Ile Pro Trp Tyr Arg His Leu Asn Arg
1               5                   10                  15
Ala Gln Trp Arg Ala Phe Ser Ala Ala Trp Leu Gly Tyr Leu Leu Asp
            20                  25                  30
Gly Phe Asp Phe Val Leu Ile Ala Leu Val Leu Thr Glu Val Gln Gly
        35                  40                  45
Glu Phe Gly Leu Thr Thr Val Gln Ala Ala Ser Leu Ile Ser Ala Ala
    50                  55                  60
Phe Ile Ser Arg Trp Phe Gly Gly Leu Met Leu Gly Ala Met Gly Asp
65                  70                  75                  80
Arg Tyr Gly Arg Arg Leu Ala Met Val Thr Ser Ile Val Leu Phe Ser
                85                  90                  95
Ala Gly Thr Leu Ala Cys Gly Phe Ala Pro Gly Tyr Ile Thr Met Phe
            100                 105                 110
Ile Ala Arg Leu Val Ile Gly Met Gly Met Ala Gly Glu Tyr Gly Ser
        115                 120                 125
Ser Ala Thr Tyr Val Ile Glu Ser Trp Pro Lys His Leu Arg Asn Lys
    130                 135                 140
Ala Ser Gly Phe Leu Ile Ser Gly Phe Ser Val Gly Ala Val Val Ala
145                 150                 155                 160
Ala Gln Val Tyr Ser Leu Val Val Pro Val Trp Gly Trp Arg Ala Leu
                165                 170                 175
Phe Phe Ile Gly Ile Leu Pro Ile Ile Phe Ala Leu Trp Leu Arg Lys
            180                 185                 190
Asn Ile Pro Glu Ala Glu Asp Trp Lys Glu Lys His Ala Gly Lys Ala
        195                 200                 205
Pro Val Arg Thr Met Val Asp Ile Leu Tyr Arg Gly Glu His Arg Ile
    210                 215                 220
Ala Asn Ile Val Met Thr Leu Ala Ala Ala Thr Ala Leu Trp Phe Cys
225                 230                 235                 240

Phe Ala Gly Asn Leu Gln Asn Ala Ala Ile Val Ala Val Leu Gly Leu
                245                 250                 255
Leu Cys Ala Ala Ile Phe Ile Ser Phe Met Val Gln Ser Ala Gly Lys
            260                 265                 270
Arg Trp Pro Thr Gly Val Met Leu Met Val Val Val Leu Phe Ala Phe
        275                 280                 285
Leu Tyr Ser Trp Pro Ile Gln Ala Leu Leu Pro Thr Tyr Leu Lys Thr
    290                 295                 300
Asp Leu Ala Tyr Asn Pro His Thr Val Ala Asn Val Leu Phe Phe Ser
305                 310                 315                 320
Gly Phe Gly Ala Ala Val Gly Cys Cys Val Gly Gly Phe Leu Gly Asp
                325                 330                 335
Trp Leu Gly Thr Arg Lys Ala Tyr Val Cys Ser Leu Leu Ala Ser Gln
            340                 345                 350
Leu Leu Ile Ile Pro Val Phe Ala Ile Gly Gly Ala Asn Val Trp Val
        355                 360                 365
Leu Gly Leu Leu Leu Phe Phe Gln Gln Met Leu Gly Gln Gly Ile Ala
    370                 375                 380
Gly Ile Leu Pro Lys Leu Ile Gly Gly Tyr Phe Asp Thr Asp Gln Arg
385                 390                 395                 400
Ala Ala Gly Leu Gly Phe Thr Tyr Asn Val Gly Ala Leu Gly Gly Ala
                405                 410                 415
Leu Ala Pro Ile Ile Gly Ala Leu Ile Ala Gln Arg Leu Asp Leu Gly
            420                 425                 430
Thr Ala Leu Ala Ser Leu Ser Phe Ser Leu Thr Phe Val Val Ile Leu
        435                 440                 445
Leu Ile Gly Leu Asp Met Pro Ser Arg Val Gln Arg Trp Leu Arg Pro
    450                 455                 460
Glu Ala Leu Arg Thr His Asp Ala Ile Asp Gly Lys Pro Phe Ser Gly
465                 470                 475                 480
Ala Val Pro Phe Gly Ser Ala Lys Asn Asp Leu Val Lys Thr Lys Ser
                485                 490                 495

<210> 6
<211> 687
<212> DNA
<213> Neisseria meningitidis

<220>
<223> CMP-sialic acid synthetase gene

<400> 6
atggaaaaac aaaatattgc ggttatactt gcgcgccaaa actccaaagg attgccatta 60
aaaaatctcc ggaaaatgaa tggcatatca ttacttggtc atacaattaa tgctgctata 120
tcatcaaagt gttttgaccg cataattgtt tcgactgatg gcgggttaat tgcagaagaa 180
gctaaaaatt tcggtgtcga agtcgtccta cgccctgcag agctggcctc cgatacagcc 240
agctctattt caggtgtaat acatgcttta gaaacaattg gcagtaattc cggcacagta 300
accctattac aaccaaccag tccattacgc acaggggctc atattcgtga agctttttct 360
ctatttgatg agaaaataaa aggatccgtt gtctctgcat gcccaatgga gcatcatcca 420
ctaaaaaccc tgcttcaaat caataatggc gaatatgccc ccatgcgcca tctaagcgat 480
ttggagcagc ctcgccaaca attacctcag gcatttaggc ctaatggtgc aatttacatt 540
aatgatactg cttcactaat tgcaaataat tgttttttta tcgctccaac caaactttat 600
attatgtctc atcaagactc tatcgatatt gatactgagc ttgatttaca acaggcagaa 660
aacattctta atcacaagga aagctaa 687

## Description

### FIELD OF THE INVENTION

The present invention relates to a method of preparing 6-thio-sialyl-glycosyl compounds, comprising the step consisting of reacting *in vitro* or *in vivo* 6-thio-sialic acid compounds with a CMP-sialic synthetase to form CMP-6-thio sialic acid compounds and with a sialyltransferase to transfer said CMP-6-thio sialic acid compounds as donor substrate to glycosyl compounds as acceptor subtrate leading to the enzymatic coupling of 6-thiosialic acid compounds onto glycosyl compounds. Glycosyl compounds are selected from glycoproteins, glycolipids and oligosaccharides, including monoclonal antibodies.

### BACKGROUND OF THE INVENTION

Sialic acids constitute a family of naturally occurring 2-keto-3-deoxy-nonulosonic acids ubiquitously displayed on cell-surface glycoconjugates (glycoproteins and glycolipids) whose *N*-acetylneuraminic acid (Neu5Ac) is the most common form found in human.' Generally displayed as a single terminal residue appended to an underlying galactose (Gal) or N-Acetylgalactosamine (GaINAc) by an α-2,3- or α-2,6-glycosidic linkage or to a sialic acid by an α-2,8-linkage, sialic acids are ideally positioned to participate in carbohydrate-protein interactions that mediate recognition phenomena. These include acting as receptors for bacteria and viruses, masking underlying antigenic determinants, mediating cell-cell adhesion via lectins, and enabling cell-cell communication processes.^{2, 3} Besides their function of modulator in cellular recognition, sialic acids strongly influence the physicochemical and biological properties of proteins to which they are attached by affecting their folding and stability.⁴ In particular, the content of sialylated glycans on a glycoprotein improves its pharmacokinetics by affecting its net charge and molecular mass. Whereas sialylation can improve plasma half-life, the presence of certain carbohydrate moieties such as galactose or N-acetylgalactosamine can trigger lectin-mediated clearance, thereby reducing plasma half-life. For example, asialylated glycoproteins are subject to receptor-mediated clearance by the asialoglycoprotein receptor (ASGPR) found in the liver upon specific binding to terminal galactosyl units.⁵ Similarily, glycoproteins with terminal mannose (Man) or N-Acetylglucosamine (GlcNAc) are cleared through the reticulo-endothelial system which relies on several receptors with high affinity for both Man and GlcNAc.⁶

Human erythropoietin (EPO) contains three *N*-linked glycans that can constitute up to 40% of the molecular mass. Although deglycosylated EPO displays threefold higher *in vitro* potency than its glycosylated counterpart, it exhibits no *in vivo* activity. Ensuring the presence of the terminal sialic acid on *N*-linked carbohydrate groups of therapeutic glycoproteins is an important consideration for their commercial development.⁷⁻⁹ Indeed, the majority of protein pharmaceuticals being developed for human therapy are glycoproteins. These include erythropoietin, granulocyte macrophage-colony-stimulating factor, and tissue plasminogen activator, which together generate sales of 3-5 billion dollars worldwide. In addition, approximately 60 recombinant glycoproteins are currently in development as therapeutic agents.

One of the most significant problems associated with pharmaceutical applications of glycoconjugates and more specifically with glycoproteins is their natural microheterogeneity. Most commonly, glycoproteins are produced in a number of forms (glycoforms) that possess the same peptide backbone, but differ in both the nature and site of glycosylation.
The glycan heterogeneity can result from underglycosylation during the production process and from degradation by glycosidases. In particular, sialidases also known as neuraminidases (EC 3.2.1.18) catalyze the removal of sialic acids from a variety of glycoconjugates.^{10, 11} Sialidases are widely distributed in nature, including viruses, bacteria, fungi, animals and humans. Perhaps the most widely reported sialidase is from the influenza virus, where it plays an important role in viral replication and release from infected cells.¹² Sialidases are produced by a wide range of bacteria and were reported as virulence factors in important diseases.¹³ Four genetically distinct forms of mammalian sialidase have been characterized, each with a predominant cellular localization (lysosomal, cytosolic or plasma membrane-associated) and substrate specificity.¹⁴ In humans, sialidases are suggested to play a role in such diverse cellular phenomena as molecular transport, antigen masking, proliferation, differentiation and membrane function.¹⁵ In particular they have clearly been implicated in two important diseases called sialidosis and galactosialidosis¹⁶ and were found to behave in different manners during carcinogenesis.¹⁷ At last, neuraminidases considerably decrease the blood stream half-life of therapeutic glycoconjugates and this problem has now become critical in the devise of effective therapeutic treatments with recombinant glycoproteins.

In connection with the present invention, we investigated procedures to enzymatically cap carbohydrate chains that lack a terminal sialic acid with a more stable NeuAc analogue.

S-Linked oligosaccharides constitute a large family of specific glycoside hydrolases inhibitors. These thiooligosaccharides which contain a glycosidic linkage resistant to enzymatic cleavage provided important structural features concerning the mechanism of this class of enzymes.¹⁸ Although small differences in S-containing oligosaccharide conformation are observed due to the longer sulphur-carbon bond compared to the oxygen-carbon bond, thiooligosaccharides have similar affinities for proteins than their naturally occurring counterparts. Potential carbohydrate-based therapeutics based on thiosugars recently received high attention. Synthetic carbohydrate vaccines incorporating thiooligosaccharides-protein conjugates have been developed by Bundle and co-workers against pathogenic bacteria Candida albicans¹⁹ and for anticancer therapy.²⁰ S-linked immunogens, as components of conjugate vaccines, clearly generate an antigen specific immune response that approaches or exceeds the response to the native oligosaccharide.²¹

However, the chemical synthesis of oligosaccharides and more specifically of thioanalogues is hampered by the several protection, deprotection and glycosylation steps resulting in low overall yields.

We search for solutions to provide enzymatic synthesis catalyzed by recombinant enzymes *in vitro* or by genetically engineered whole cells which have emerged as efficient approaches for the large scale preparation of oligosaccharides.²²

Thiooligosaccharides have already been prepared by enzymatic approaches either by use of glycosyltransferases or mutant glycoside hydrolases (thioglycoligases and thioglycosynthases).²³ In S-linked oligosaccharides, the glycosidic acceptor part is chemically modified by a sulphur atom at the branch point prior to enzymatic condensation. Glucose, Mannose and Glucuronic acid residues have been coupled to S-modified glucoside and xyloside.^{24, 25} The thioligase approach allowed Withers and coworkers the glycosidic remodelling of a recombinant protein, necessiting the site specific chemical ligation of the thiol acceptor prior to enzymatic *S*-galactosylation.²⁶ Few glycosyltransferases have also been shown to promote *S*-glycosylation on chemically modified acceptors. Recombinant α-1,3-Galactosyltransferase and β-1,3-Glucosaminyltransferase from *Neisseria meningitidis* afforded expected *S*-linked products²⁷ whereas wild type sialyltransferases failed to transfer sialic acid onto 3'S-lactose.²⁰ Withers and coworkers recently obtained by directed evolution a mutant sialyltransferase able to performe this last reaction on an hydrophobic derivative of 3'S-lactose. There is however no indication that such a mutant will be suitable for protein remodellin.²⁸

Thiooligosaccharides in which the sulphur atom is incorporated in the carbohydrate ring in place of the oxygen atom present the same potential as *S*-linked compound.²⁹ Several mono, di and oligosaccharidic analogues incorporating 5-*S*-Glc, Man, Fuc, Xyl have been prepared and used in glycosidase,³⁰ lectin^{31, 32} and antibody³³ inhibition assays. For example, 5-thioxylopyranosides compounds are developed by Fournier Pharma as active principle in medicaments that are intended for the treatment or prevention of thromboses or heart failure.^{34,35} 5'-Thio-*N*-acetyllactosamine prepared by Hindsgaul and coworkers through chemical-enzymatic synthesis showed 200-fold increased stability to digestion by a galactosidase.³⁶ The synthesis of 6-*S*-Neu5Ac and analogues has been reported in 1987 by Mack & Brossme^{37,38} and in 1996 by von Itzstein and coworkers³⁹ for the development of influenza virus inhibitors. Although GDP-5-S-Fuc, GDP-5-*S*-Man⁴⁰ and UDP-5-*S*-Gal³⁶ are effective substrate analogues for appropriate glycosyltransferases, no report of enzymatic sialylation of 6-*S*-Neu5Ac has been reported so far in the literature.

We discovered that 6-S-Sialic acid compounds, such as compounds of formula I below, can effectively be activated in their CMP form and be accepted as substrate by sialyltransferases and transferred into the terminal residue of oligosaccharides. Thus, the present invention provides for the first time *in vitro* and *in vivo* methods to transfer 6-S-Sialic acids onto suitable glycosylated acceptors including glycoproteins.

### DESCRIPTION

The present invention relates to a method of enzymatic coupling of 6-thiosialic acid compounds onto glycosyl compounds, including glycoproteins, by a growing metabolically engineered microorganism expressing a CMP-sialic synthetase and a sialyltransferase, such as a metabolically engineered *E*. *coli,* or by *in vitro* synthesis using a crude enzymatic extract comprising a CMP-sialic synthetase and a sialyltransferase.

As used herein, the term "6-thio-sialic acid compounds" means 6-thio-neuraminic acid (5-amino-2,6-anhydro-3,5-dideoxy-6-thio-D-glycero-α,β-D-galacto-2-non-ulosonic acid), and derivatives such as an *N-* or *O*-acyl derivative of neuraminic acid. Preferably, the 6-thio-sialic acid is the N-acetyl derivative of neuraminic acid reported by Kok *et al.*³⁹ and *Mack et al..*^{37,} 38 Preferably, 6-thio-sialic acid derivatives according to the invention are of formula I depicted below with possible substitutions at the carbon atom at positions 4, 5, 7, 8 or 9 : 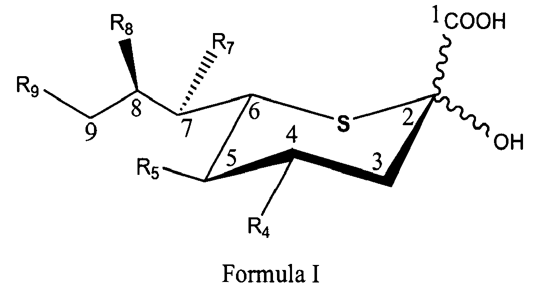 Wherein
R4 is OH, *O*-acetyl, *O*-galactosyl, *O*-fucosyl, or N₃
R5 is NH₂, OH, N₃, NHCOR' (R'= CH₃, CH₂Ph, CH₂N₃, CH=CHCH₃, CH₂CH₂COCH₃, (CH₂)ₙCH₃ where n is ≥1, or L-OCH₂CH₂(OCH₂CH₂)ₙOCH₃) wherein L is a substituted or unsubstituted alkyl or heteroalkylgroup.
R7 is OH, *O*-acetyl, or NHCOR' ; R' being defined as above
R8 is OH, *O*-acetyl, *O*-methyl, sulfate, *O*-sialyl, or *O*-glucosyl
and R9 is OH, *O*-acetyl, *O*-lactyl, phosphate, *O*-sialyl, F, or N₃

Particularly preferred sialic acids are 6-thio-Neu5Ac (R_{4,7,8,9} = OH, R₅ = NHAc), 6-Thio-*N-*glycolylneuraminic acid (R_{4,5,7,8,9} = OH) and 6-thio-*N*-acyl-neuraminic acid (R_{4,7,8,9} = OH, R₅= NHCOR' where R' is a substituted or unsubstituted alkyl or heteroalkyl chain).

In this method, the 6-S-sialic acid is activated into cytidine 5'-monophosphate 6-S-sialic acid by an *N*-acylneuraminate cytidylyltransferase also known as CMP-sialic synthetase (EC-number 2.7.7.43) before being transferred onto a glycosyl acceptor by a sialyltransferase in a one pot sequence.

CMP-sialic acid synthetase catalyzes the formation of CMP-sialic acid from sialic acid and commercially available or endogenous *E. coli* cytidine 5'-triphosphate (CTP). CMP-sialic acid synthetase used in accordance to the present invention can be purchased from Calbiochem, QA-BIO LLC or isolated and purified from cells and tissues containing the synthetase enzyme by procedures well known in the art.^{47, 48} In a preferred embodiment, the CMP sialic acid synthase is from *Neisseria meningitidis*⁴⁹ and is produced in *E*. *coli.*^{50, 51} It can also be encoded by the *neuA* gene from *C. jejuni* strain ATCC accession number 43438.

The above method also allows the transfer of the CMP 6-S-sialic acid onto a glycosyl compound by an appropriate sialyltransferase. Sialyltransferases (STs) are a family of glycosyltransferases that catalyze the transfer of sialic acid residues from CMP-sialic acids as donor substrates to the carbohydrate groups of glycoproteins and glycolipids as acceptor substrates according to the following reaction:

CMP-**sialic acid** + HO-R --> CMP + **sialyl**-O-R

Twenty distinct sialyltransferases have been identified in both human and murin genomes.^{52,} 53 Each of the sialyltransferase genes is differentially expressed in a tissue-, cell type-, and stage-specific manner to regulate the sialylation pattern of cells. These enzymes differ in their substrate specificity, tissue distribution and various biochemical parameters. However, enzymatic analysis conducted *in vitro* with recombinant enzyme revealed that one linkage can be synthesized by multiple enzymes. Sialyltransferases are not unique to mammals, they have also been documented in bacteria,⁵⁴ fish, birds and amphibians.⁵⁵

Sialyltransferases can be divided into three broad classes according to the regioselectivity of sialic acid attachment to the substrate acceptor. Under the nomenclature proposed by Tsuji et al.,⁵⁶ theses families are designated ST3, ST6, and ST8 to denote enzymes that transfer sialic acids to the 3-OH, 6-OH or 8-OH hydroxyl positions of the underlying carbohydrate respectively.

So, depending on the acceptor, one may choose the appropriate sialyltransferase as for example listed below in Table I:

| Name | Other name | Preferred acceptor^{a} |
|---|---|---|
| **2,3-STs** | | |
| ST3Ga1 I | ST-3O, ST3GalA.1, ST-2 | **Gal**β1,3GalNAc |
| ST3Gal II | ST3GalA.2, SAT-IV | **Gal**β1,3GalNAc |
| ST3Gal III | ST-3N, ST-3 | **Gal**β1,3GlcNAc |
| ST3Gal IV | ST-Z, SAT-3, ST-4 | **Gal**β1,4GlcNAc |
| | | |

| **2,6-STs** | | |
|---|---|---|
| ST6Gal I | ST-6N, SiaT-1, ST-1 | **Gal**β1,4GlcNAc |
| ST6GalNAc I | ST3O-I | **GalNAc**αSer/Thr |
| | | Galβ1,3**GalNAc**αSer/Thr |
| | | Siaα2-3Galβ1,3**GalNAc**αSer/Thr |
| ST6GalNAc II | | Siaα2-3Galβ1,3**GalNAc**αSer/Thr |
| | | Galβ1,3**GalNAc**αSer/Thr |
| ST6GlcNAc III | STY, ST60-II | Siaα2-3Galβ1,3**GalNAc**αSer/Thr |
| ST6GlcNAc | | (Siaα2-3Galβ1-3)**GlcNAc**βR |
| | | |

| **2,8-STs** | | |
|---|---|---|
| ST8Sia I | SAT-II (SAT-III), GD3 synthase | GM3 (a-series) |
| | | |
| ST8Sia II | STX | glycoprotein |
| ST8Sia III | | Siaα2-3Galβ1,4GlcNAc |
| | | GD3 (b-series) |
| ST8Sia IV | PST-1 | glycoprotein |
| ST8Sia V | SAT-V/-SAT-III | GD1a, GT1b, GD3 |

| | | |
|---|---|---|
| ^{a} Shown in bold is the sugar residue to which the sialic acid is attached Table extract from ref⁵⁷ | | |

All known ST3 enzymes transfer sialic acids to 3-OH of galactose residue in acceptor glycans. Biosynthesis of the α-2,6-sialyl linkage to Gal, GalNAc, or GlcNAc is mediated by the α-2,6 STs. α-2-8-Linked polysialic glycotopes occur in glycoproteins from a variety of living species ranging from bacteria to human.⁵⁸ Particularly, polySia chains were shown to be expressed on the embryonic form of neural cell adhesion molecule (N-CAM) in developmentally regulated manner.⁵⁹

Although sialyltransferases can be isolated and purified from animal tissues, expression of recombinant forms in bacteria or yeast are more adapted to synthetic purposes. Bacterial sialyltransferases which are efficiently expressed on large scale in *E*. *coli* were shown to exhibit broader substrate specificity than that of mammalian counterparts conferring potential usefulness for enzymatic synthesis of natural and nonnatural sialoglycoconjugates.⁵⁴ Although yeast cells do not naturally synthesize sialoglycoconjugates, expression of mammalian sialyltransferases through metabolic engineering resulted in the synthesis of sialylated *N*-glycans. In particular, active α-2,6- and 2,3-sialyltransferases have been respectively expressed in *Pichia pastoris*⁶⁰ and *Saccharomyces cerevisiae.* ⁶¹

In a preferred embodiment, α-2,3-sialytransferase from *Neisseria meningitidis* that transfer Neu5Ac onto galactosyl acceptors is expressed in *E*. *coli*.⁵⁰. (strain MC58 (NRCC 4728), NCBI U60660 - SEQ ID No 4).

In another embodiment, bifunctional sialyltransferases are used in the methods of the invention. Nucleic acids encoding such enzymes have been isolated from *C*. *jejuni* and are disclosed in U.S. Patent Nos. 6,699,705 and 6503744 and WO/02074942. Exemplary *C*. *jejuni* strains which can be used as sources of bifunctional sialyltransferases include OH4384 (sequences are found in GenBank accessions AR271700 and AX934425 - SEQ ID No 1), OH4382, 0:10 (sequences are found in GenBank accessions AX934427 - SEQ ID No 2), O:23, and O:41 (nucleic acid sequences are found in GenBank accessions AX934429 - SEQ ID No 3). It shall be understood that conservatively modified variations as defined above of SEQ ID No 1, 2, 3 may be applied herein.

The present invention provides a method to introduce a 6-thio-sialic acid onto a glycosyl acceptor with a sialyltransferase. As used herein, the term "a glycosyl compound" refers to an organic compound having one or more glycosyl residues. A "glycosyl residue" is a cyclic monosaccharide or monosaccharide derivative lacking the anomeric hydroxyl group. Preferred glycosyl residues are galactosyl (Gal), *N*-acetylglucosyl (GlcNAc), *N-*acetylgalactosyl (Ga1NAc) and sialyl (Sia). The glycosyl residue acts as the acceptor site for the sialic acid, and therefore must have an appropriate hydroxyl group available to accept the sialic acid group. Acceptor glycosyl residues can exist as monosaccharides, oligosaccharides (containing from 2 to 9 monosaccharides), polysaccharides (containing 10 or more monosaccharides), glycopeptides, glycoproteins and glycolipids.

Thus, in a first embodiment, the invention is directed to a method of preparing 6-thio-sialyl-glycosyl compounds, comprising the step consisting of reacting *in vitro* or *in vivo* 6-thio-sialic acid compounds selected from 6-thio-sialic acid, *N*- or *O*-acyl derivative thereof or a derivative of formula I as defined above with a CMP-sialic synthetase to form CMP-6-thio sialic acid compounds and with a sialyltransferase to transfer said CMP-6-thio sialic acid compounds as donor substrate to glycosyl compounds as acceptor subtrate leading to the enzymatic coupling of 6-thiosialic acid compounds onto glycosyl compounds.

Glycosyl compounds are as defined above and can be selected particularly from glycoproteins, glycolipides and oligosaccharides.

For example, the method described above allows the sialylation with 6-thio-sialic acid of glycoproteins harbouring glycans terminated by a Gal, GalNAc, GlcNac or Sia residue. Prefered glycoproteins are those harbouring complex (human) type glycans.
Glycoproteins can be selected from Erythropoietin (EPO), Granulocyte colony-stimulating factor (G-CSF), DNAse, Tissue plasminogen activator (TPA), Glucocerebrosidase, Interferon such as Interferon-beta-la, Interferon beta-1b, Insulin-like growth factor 1, Human Growth Hormon (HGH), human insulin, Follicle-stimulating hormone FSH, Factor VII and VIII.

Glycoproteins can be selected from monoclonal antibodies, such as for example anti-CD3 to prevent acute rejection of organ, anti-TNF-α to treat some inflammatory diseases such as rheumatoid arthritis, anti-IgE against allergic asthma, anti-IL-2 receptor to prevent acute rejection of transplanted kidneys and to treat T-cell lymphoma, anti-CD20 to treat B-cell lymphomas, anti-epidermal growth factor (EGF) receptor found on some tumor cells, anti-CD33, a cell-surface molecule expressed by the cancerous cells in acute myelogenous leukemia (AML), anti-CD22 for some B-cell leukemias, anti-CD52 to treat chronic lymphocytic leukemia, anti-HLA-DR for lymphomas, and anti-VEGF.

For example, glycoproteins comprises glycans comprising biantennary GlcNac2Man3Gal(0, 1, or 2) structures with or without fucose.
Examples of such structures are: 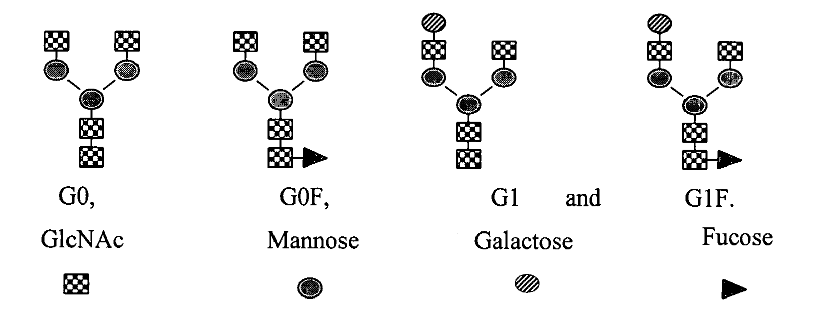 and

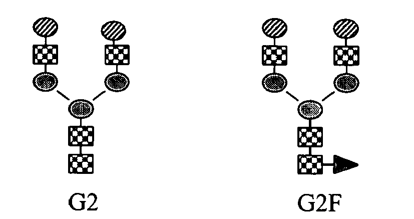

On the above structure, the invention allows the addition of one or two 6-thio-sialic acid compound as defined above

So, it within the scope of the invention to provide such glycoproteins harbouring 6-thio-sialic acid, *N*- or *O*-acyl derivative thereof or a derivative of formula I, for example humanized glycoproteins wherein sialic acid residue(s) is replaced by 6-thio-sialic acid, *N-* or *O*-acyl derivative thereof or a derivative of formula I.
Such glycoproteins can be produced by different expression systems such as for example mammalian cells (i.e Chinese hamster ovary cells CHO), insect cells, yeasts (*P. pastoris, Saccharomyces cerevisae*) or bacteria (*E. coli*).

The recombinant cells of the invention are generally made by creating or otherwise obtaining a polynucleotide that encodes the particular enzyme(s) of interest, placing the polynucleotide in an expression cassette under the control of a promoter and other appropriate control signals, and introducing the expression cassette into a cell. More than one of the enzymes can be expressed in the same host cells using a variety of methods. For example, a single extrachromosomal vector can include multiple expression cassettes or more than one compatible extrachromosomal vector can be used maintain an expression cassette in a host cell. Expression cassettes can also be inserted into a host cell chromosome, using methods known to those of skill in the art. Those of skill will recognize that combinations of expression cassettes in extrachromosomal vectors and expression cassettes inserted into a host cell chromosome can also be used. Other modification of the host cell, described in detail below, can be performed to enhance production of the desired oligosaccharide. The recombinant cells of the invention are generally microorganisms, such as, for example, yeast cells, bacterial cells, or fungal cells. Examples of suitable cells include, for example, *Azotobacter sp.* (*e.g., A. vinelandii*), *Pseudomonas sp., Rhizobium sp., Erwinia sp., Bacillus sp., Streptomyces sp., Escherichia sp.* (*e.g., E. coli*)*,* and *Klebsiella sp.,* among many others. The cells can be of any of several genera, including Saccharomyces (*e.g., S. cerevisiae*), Candida (*e.g., C. utilis, C. parapsilosis, C. krusei, C. versatilis, C. lipolytica, C. zeylanoides, C. guilliermondii, C. albicans, and C. humicola*), Pichia (*e.g., P. farinosa and P. ohmeri*), Torulopsis (*e.g., T. candida, T. sphaerica, T. xylinus, T. famata, and T. versatilis*), Debaryomyces (*e.g., D. subglobosus, D. cantarellii, D. globosus, D. hansenii, and D. japonicus*), Zygosaccharomyces (*e.g., Z. rouxii and Z. bailii*), Kluyveromyces (*e.g., K. marxianus*), Hansenula (*e.g., H. anomala and H. jadinii*), and Brettanomyces (*e.g., B. lambicus and B. anomalus*).

Promoters for use in *E. coli* include the T7, trp, or lambda promoters. A ribosome binding site and preferably a transcription termination signal are also provided. For expression of heterologous proteins in prokaryotic cells other than *E. coli,* a promoter that functions in the particular prokaryotic species is required. Such promoters can be obtained from genes that have been cloned from the species, or heterologous promoters can be used. For example, the hybrid trp-lac promoter functions in *Bacillus* in addition to *E. coli.* Methods of transforming prokaryotes other than *E. coli* are well known. For example, methods of transforming *Bacillus* species and promoters that can be used to express proteins are taught in U.S. Patent No. 6,255,076 and U.S. Patent No. 6,770,475.

In yeast, convenient promoters include GAL1-10 (Johnson and Davies (1984) Mol. Cell. Biol. 4:1440-1448) ADH2 (Russell et al. (1983) J. Biol. Chem. 258:2674-2682), PHO5 (EMBO J. (1982) 6:675-680), and MFα (Herskowitz and Oshima (1982) in The Molecular Biology of the Yeast Saccharomyces (eds. Strathern, Jones, and Broach) Cold Spring Harbor Lab., Cold Spring Harbor, N.Y., pp. 181-209). Another suitable promoter for use in yeast is the ADH2/GAPDH hybrid promoter as described in Cousens et al., Gene 61:265-275 (1987). For filamentous fungi such as, for example, strains of the fungi *Aspergillus* (McKnight *et al*., U.S. Patent No. 4,935,349), examples of useful promoters include those derived from *Aspergillus nidulans* glycolytic genes, such as the ADH3 promoter (McKnight et al., EMBO J. 4: 2093 2099 (1985)) and the *tpiA* promoter. An example of a suitable terminator is the ADH3 terminator (McKnight *et al*.).

In some embodiments, the polynucleotides are placed under the control of an inducible promoter, which is a promoter that directs expression of a gene where the level of expression is alterable by environmental or developmental factors such as, for example, temperature, pH, anaerobic or aerobic conditions, light, transcription factors and chemicals. Such promoters are referred to herein as "inducible" promoters, which allow one to control the timing of expression of the glycosyltransferase or enzyme involved in nucleotide sugar synthesis. For *E. coli* and other bacterial host cells, inducible promoters are known to those of skill in the art. These include, for example, the *lac* promoter. A particularly preferred inducible promoter for expression in prokaryotes is a dual promoter that includes a *tac* promoter component linked to a promoter component obtained from a gene or genes that encode enzymes involved in galactose metabolism (*e.g*., a promoter from a UDPgalactose 4-epimerase gene (galE)). The construction of polynucleotide constructs generally requires the use of vectors able to replicate in bacteria. A plethora of kits are commercially available for the purification of plasmids from bacteria. For their proper use, follow the manufacturer's instructions (see, for example, EasyPrepJ, FlexiPrepJ, both from Pharmacia Biotech; StrataCleanJ, from Stratagene; and, QIAexpress Expression System, Qiagen). The isolated and purified plasmids can then be further manipulated to produce other plasmids, and used to transfect cells. Cloning in *Streptomyces* or *Bacillus* is also possible.

Selectable markers are often incorporated into the expression vectors used to construct the cells of the invention. These genes can encode a gene product, such as a protein, necessary for the survival or growth of transformed host cells grown in a selective culture medium. Host cells not transformed with the vector containing the selection gene will not survive in the culture medium. Typical selection genes encode proteins that confer resistance to antibiotics or other toxins, such as ampicillin, neomycin, kanamycin, chloramphenicol, or tetracycline. Alternatively, selectable markers may encode proteins that complement auxotrophic deficiencies or supply critical nutrients not available from complex media, e.g., the gene encoding D-alanine racemase for Bacilli. Often, the vector will have one selectable marker that is functional in, *e.g*., *E. coli,* or other cells in which the vector is replicated prior to being introduced into the target cell. A number of selectable markers are known to those of skill in the art and are described for instance in Sambrook *et al.* A preferred selectable marker for use in bacterial cells is a kanamycin resistance marker (Vieira and Messing, *Gene* 19: 259 (1982)). Use of kanamycin selection is advantageous over, for example, ampicillin selection because ampicillin is quickly degraded by β-lactamase in culture medium, thus removing selective pressure and allowing the culture to become overgrown with cells that do not contain the vector.

Construction of suitable vectors containing one or more of the above listed components employs standard ligation techniques as described in the references cited above. Isolated plasmids or DNA fragments are cleaved, tailored, and re-ligated in the form desired to generate the plasmids required. To confirm correct sequences in plasmids constructed, the plasmids can be analyzed by standard techniques such as by restriction endonuclease digestion, and/or sequencing according to known methods. Molecular cloning techniques to achieve these ends are known in the art. A wide variety of cloning and *in vitro* amplification methods suitable for the construction of recombinant nucleic acids are well-known to persons of skill.

A variety of common vectors suitable for constructing the recombinant cells of the invention are well known in the art. For cloning in bacteria, common vectors include pBR322 derived vectors such as pBLUESCRIPT^{™}, and λ-phage derived vectors. In yeast, vectors include Yeast Integrating plasmids (*e.g*., YIp5) and Yeast Replicating plasmids (the YRp series plasmids) and pGPD-2.

The methods for introducing the expression vectors into a chosen host cell are not particularly critical, and such methods are known to those of skill in the art. For example, the expression vectors can be introduced into prokaryotic cells, including *E*. *coli,* by calcium chloride transformation, and into eukaryotic cells by calcium phosphate treatment or electroporation. Other transformation methods are also suitable.

A "heterologous polynucleotide" or a "heterologous gene", as used herein, is one that originates from a source foreign to the particular host cell, or, if from the same source, is modified from its original form. Thus, a heterologous sialyltransferase gene in a cell includes a gene that is endogenous to the particular host cell but has been modified. Modification of the heterologous sequence may occur, e.g., by treating the DNA with a restriction enzyme to generate a DNA fragment that is capable of being operably linked to a promoter. Techniques such as site-directed mutagenesis are also useful for modifying a heterologous sequence.

A "recombinant expression cassette" or simply an "expression cassette" is a nucleic acid construct, generated recombinantly or synthetically, with nucleic acid elements that are capable of affecting expression of a structural gene in hosts compatible with such sequences. Expression cassettes include at least promoters and optionally, transcription termination signals. Typically, the recombinant expression cassette includes a nucleic acid to be transcribed (*e.g*., a nucleic acid encoding a desired polypeptide), and a promoter. Additional factors necessary or helpful in effecting expression may also be used. Transcription termination signals, enhancers, and other nucleic acid sequences that influence gene expression, can also be included in an expression cassette. When more than one heterologous protein is expressed in a microorganism, the genes encoding the proteins can be expressed on a single expression cassette or on multiple expression cassettes that are compatible and can be maintained in the same cell. As used herein, expression cassette also encompasses nucleic acid constructs that are inserted into the chromosome of the host microorganism. Those of skill are aware that insertion of a nucleic acid into a chromosome can occur, e.g., by homologous recombination. An expression cassette can be constructed for production of more than one protein. The proteins can be regulated by a single promoter sequence, as for example, an operon. Or multiple proteins can be encoded by nucleic acids with individual promoters and ribosome binding sites.

In the method as depicted above, the enzymatic coupling of 6-thiosialic acid compounds onto glycosyl compounds is performed using a metabolically engineered microorganism expressing a CMP-sialic synthetase and a sialyltransferase. Alternatively, the enzymatic coupling of 6-thiosialic acid compounds onto glycosyl compounds is performed *in vitro* using a crude enzymatic extract or is performed *in vitro* using purified CMP-sialic synthetase and sialyltransferase.

In one specific embodiment, 6-thio sialic acid compounds are 6-thio-Neu5Ac (R_{4,7,8,9} = OH, R₅ = NHAc), 6-Thio-*N*-glycolylneuraminic acid (R_{4,5,7,8,9} = OH) or 6-thio-*N*-acyl-neuraminic acid (R_{4,7,8,9} = OH, R₅= NHCOR' where R' is a substituted or unsubstituted alkyl or heteroalkyl chain). Also, CMP sialic acid synthase can be from *Neisseria meningitidis* such as strain 406Y (NRCC 4030) NCBI U60146 - SEQ ID No 6 or is encoded by a *neuA* gene, such as the *neuA* from *C. jejuni* strain ATCC accession number 43438.

In one preferred embodiment, the method can be practiced to transfer 6-thio sialic acid compounds as depicted above onto galactosyl acceptors. But, more generally, the method of the invention is useful for the sialylation with 6-thio-sialic acid of glycoproteins harbouring glycans terminated by a Gal, GalNAc, GlcNac or Sia residue, such as glycoproteins comprising glycans comprising biantennary GlcNac2Man3Gal(0, 1, or 2) structures with or without bisecting GlcNac as shown above.

In another particular aspect, the invention concerns a method as described above for transfering a 6-thio sialic acid compound onto glycoproteins obtained in genetically engineered yeasts which comprises alpha 1,6-mannosyl-transferase inactivation and expression of at least one of heterologous mannosidases, glycosyltransferases, galactosyl transferases and/or sialyltransferases. For example, said glycoproteins obtained from genetically engineered yeasts are incubated *in vitro* with a CMP-sialic synthetase and a sialyltransferase to introduce said 6-thio-sialic acid compound. Alternatively, the invention is aimed at a method for producing 6-thio sialylated glycoproteins in genetically engineered yeasts which comprises alpha 1,6-mannosyl-transferase inactivation and expression of at least one of heterologous mannosidases, glycosyltransferases, galactosyltransferases and/or sialyltransferases, said yeast being further modified to express an heterologous gene encoding a sialyl transporter, such as for example a nanT gene; said yeasts being cultured in a culture medium comprising said 6-thio-sialic acid compounds.

*Pichia pastoris* was recently engineered by Gerngross and coworkers (GlycoFi Inc.) to secrete human glycoproteins with fully complex terminally sialylated *N*-glycans.⁶² After the knockout of four genes to eliminate yeast-specific glycosylation, 14 heterologous genes were introduced, allowing to replicate the sequential steps of human glycosylation. The reported cell lines produce complex glycoproteins with greater than 90% terminal sialylation.

In one aspect, the invention relates to transferring a 6-thio sialic acid as defined above onto glycoproteins obtained in genetically engineered yeasts as described by Gerngross and coworkers (GlycoFi Inc.). One can choose a yeast with alpha 1,6-mannosyltransferase inactivation, expressing mannosidases, glycosyltransferases and galactosyltransferases depending on the glycoproteins to be produced. Also, a recombinant protein produced in yeasts as described by Gerngross can be incubated with a sialyltransferase to introduce said 6-thio-sialic acid as defined above. This can be performed by incubation with cell extract comprising a CMP-sialic synthetase and a sialyltransferase or a purified CMP-sialic synthetase and sialyltransferase.

Then, it is within the scope of the invention to culture yeasts producing a recombinant glycoprotein with complex type glycans and futher expressing a heterologous sialyl transporter in a culture medium comprising said 6-thio-sialic acid as defined above.

The same embodiment can be applied with CHO or any other mammalian cells expressing at least one glycosyltransferase. Thus, the invention is also aimed at a method for producing 6-thio sialylated glycoproteins in genetically modified mammalian cells, such as CHO, which comprises the expression of at least one of heterologous glycosyltransferase, such as a galactosyl transferase, and a sialyltransferase, said mammalian cells being further modified to express an heterologous gene encoding a sialyl transporter, such as for example a *nanT* gene; and being cultured in a culture medium comprising said 6-thio-sialic acid compounds.

In another preferred example, the 6-thio-sialic acid is incorporated in the protein GlycoPegylation process described by Neose Technology Inc.⁶³ Covalent attachment of polyethylene glycol, PEGylation, has been shown to prolong the half-life and enhance the pharmacodynamics of therapeutic proteins. The strategy developed by Neose relies on site-directed PEGylation using glycosyltransferases to attach PEG to O-glycans. The process involves enzymatic GalNAc glycosylation at specific serine and threonine residues in proteins expressed without glycosylation in *Escherichia coli*, followed by enzymatic transfer of sialic acid conjugated with PEG to the introduced GalNAc residues. The strategy was applied to three therapeutic polypeptides, granulocyte colony stimulating factor (G-CSF), interferon-alpha2b (IFN-α2b), and granulocyte/macrophage colony stimulating factor (GM-CSF), which are currently in clinical use.

In still another specific embodiment, the glycosyl acceptor can be a mono-, oligo or polysaccharide whose reducing end is functionalized by a chemical group allowing subsequent chemical or enzymatic conjugation to another chemical species (solid support, protein, oligonucleotide, peptide).

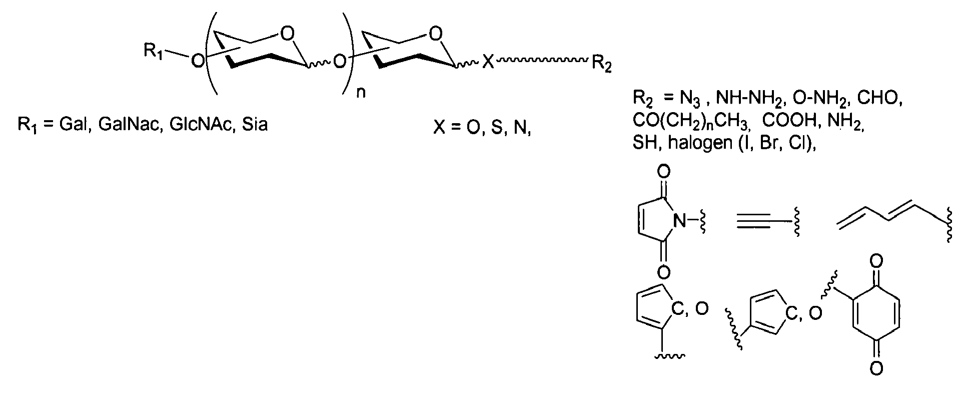

Preferred bioconjugation reactions⁶⁴ include cycloaddition reactions such as 1,3-cyclodipolar addition ("Click chemistry") between azide and alkyne groups catalyzed by Cu (I) or Diels Alder reactions.
Also preferred are nucleophilic addition reactions between amine, hydroxylamine, hydrazine and aldehyde or activated carboxylic acid as well as between thiol and maleimide or halogen.

In the example below, the glycosyl acceptor is allyl β-lactoside (X = O, R2 = CH₂CH=CH₂). This terminal alkene group was previously shown to allow specific chemical ligation reactions onto protein or other chemical species either through ozonolysis reaction affording a reactive aldehyde function or through activation into a primary amine by radical addition of cysteamine hydrochloride.⁶⁵

In this regard, a recombinant protein is first reacted with an acceptor oligosaccharide as shown above and then is incubated with a sialyltransferase to introduce said 6-thio-sialic acid as defined above. This can be performed by incubation with cell extract comprising a CMP-sialic synthetase and a sialyltransferase or a purified CMP-sialic synthetase and sialyltransferase.

In still another preferred embodiment, the above method is performed *in vivo,* comprising the step consisting of culturing a microorganism, more particularly a non pathogenic bacteria, in a culture medium comprising said 6-thio-sialic acid compounds, wherein said microorganism comprises heterologous genes encoding a CMP-Neu5Ac synthetase, and a sialyltransferase, said microorganism expressing a sialic acid transporter, such as NanT, and a beta-galactoside permease (LacY); and wherein the endogenous genes coding for sialic acid aldolase (*NanA*) and optionally for ManNac kinase (*NanK*) have been deleted or inactivated.

NanT is meant to refer to any sialic acid transporter and can be chosen for example from sialic acid transporter of *Escherichia coli* K12 NCBI ACCESSION NP_417691.4 (SEQ ID No 5).

Said microorganism is preferably LacZ- and is grown on glycerol as carbon source. Said micoorganism may further express a recombinant protein or peptide, or a monoclonal antibody.

In this aspect, the heterologous sialyltransferase gene can be selected from α-2,3-sialyltransferase, α-2,3- and α-2,8-sialyltransferase (*cstII*), and α-2,6 sialyltransferase, and the heterologous CMP-Neu5Ac synthetase can be a *neuA* gene isolated from bacterial strains that contain sialylated structure in their cells envelope, such as *C*. *jejuni* strain ATCC Accession No. 43438.

Among microorganisms, it is convenient to use an *E. coli* strain which is *LacY*+ (β-galactoside permease), *LacZ*- (β galactosidase), and optionally *MelA*- (α-galactosidase).

In this embodiment, the glycosyl acceptor is synthesized internally starting from an exogenous precursor added in the culture medium selected from lactose, galactose, β-galactoside, and α-galactoside.

The invention also relates to a microorganism or a cell as defined above and to a cell culture medium comprising a 6-thio-sialic acid compound as defined in claim 1 and the microorganism as depicted herein.

The invention is also aimed at a 6-thio-sialylated recombinant glycoprotein or antibody obtainable by the method described above and at a pharmaceutical composition comprising as active ingredient a 6-thio-sialylated recombinant glycoprotein or antibody obtainable by the method described above.

The invention also contemplates the use of a sialyltransferase to transfer a 6-thio-sialic acid compound as defined herein onto a glycosyl acceptor as defined above.

The invention is further embodied in the examples below and it will be refered to the figure legends.

### Figure legends

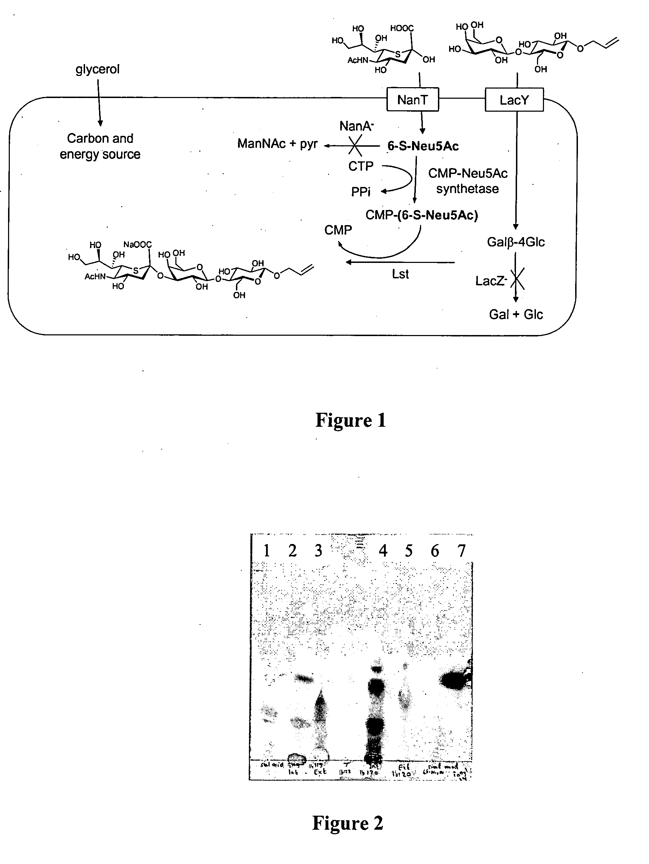

**Figure 1: Metabolically engineered pathway of allyl (6-thio-Neu5Ac)-α2,3-β-lactoside in Escherichia coli K12.**
   Allyl lactoside and 6-*S*-Neu5Ac, which are internalized by the specific permeases LacY and NanT, cannot be degraded because of β-galactosidase (LacZ) and aldolase (NanA) inactivation. 6-*S*-Neu5Ac is converted into a nucleotide-activated form CMP-(6-*S*-Neu5Ac) by CMP-Neu5Ac synthetase and then transferred onto lactose by α-2,3-sialyltransferase (encoded by Lst), to form allyl 3'(6-thio-sialyl)-β-lactoside. CTP, cytidine triphosphate; Ppi, inorganic pyrophosphate.
**Figure 2: TLC analysis (BuOH, AcOH, H₂O v/v 2/1/1) of the crude intracellular fraction revealed the presence of a new compound**
   1^{st}lane: Neu5Ac
   2^{nd} lane: intracellular fraction of control reaction (Neu5Ac+allyl lactoside)
   3^{rd} lane: extracellular fraction of control reaction (Neu5Ac+allyl lactoside)
   4^{th} lane: intracellular fraction of reaction (6-*S*-Neu5Ac+allyl lactoside)
   5^{th} lane: extracellular fraction of reaction (6-*S*-Neu5Ac+allyl lactoside)
   6^{th} lane: 6-*S*-Neu5Ac, not revelated by this TLC staining reagent (orcinol/H₂SO₄)
   7^{th} lane: allyl lactoside
**Figure 3: column chromatography on silica gel**
   1^{st} lane: allyl lactoside
   2^{nd} and 3^{rd} lane: purified intracellular fraction
   4^{th} lane: 6-*S*-Neu5Ac, not visible
**Figure 4: identification of [(5-*N*-Acetyl-6-deoxy-6-thio-α-neuraminyl)-(2→ 3)]-β-D-galactopyranosyl-(1→ 4)-β-D-glucopyranoside production**

### Example 1: Synthesis of Allyl [(5-N-acetyl-6-deoxy-6-thio-α-neuraminyl)-(2→ 3)]-β-D-galactopyranosyl-(1→ 4)-β-D-glucopyranoside by genetically modified E. coli cells either by in vivo process.

Allyl [(5-*N*-acetyl-6-deoxy-6-thio-α-neuraminyl)-(2→ 3)]-[β-D-galactopyranosyl-(1→ 4)-β-D-glucopyranoside has the following formula :

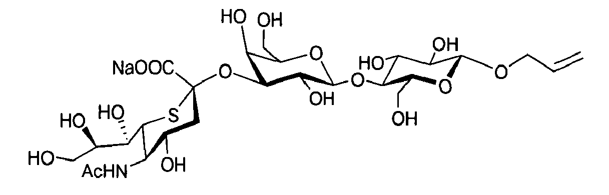

Basically, the synthesis of this compound is achieved using as starting point the process described by Samain *et al.* [Samain, E.; Priem, B. Method for producing oligopolysaccharides. WO 01/04341 (2001); B. Priem, M. Gilbert, W. W. Wakarchuk, A. Heyraud, E. Samain, Glycobiology 2002, 12, 235] with *E. coli* JM107-nanA-(Nst-01, pBBnsy) strain using allyl β-lactoside [H. B. Mereyala, S. R. Gurrala, Carbohydr. Res., 1998, 307, 351] and 6-thio-sialic acid [G. B. Kok, M. Campbell, B. Mackey, M. von Itzstein, J. Chem. Soc. Perkin Trans. 1, 1996, 2811-2815] acid as exogeneous acceptors. This strain has been further modified to allow transport of 6-thio-sialic acid compounds and the endogenous genes coding for sialic acid aldolase (*NanA*) has been inactivated.

*E. coli* JM107-nanA-(Nst-01, pBBnsy) strain is grown at low cell density culture in shake flask fermentation.

### 1.1 Growth of colonies

Agar plates consisted of LB, ampicillin (50mg/mL) and tetracyclin (15mg/mL). *E. coli* JM107-nanA-(Nst-01, pBBnsy) cells were streaked onto the plate and incubated at 37 °C for 24 h. Only single bacterial colony was selected for preculture preparations.

Minimum glycerol medium consisted of:
glycerol 7.5 g/L
NH₄H₂PO₄ 7g/L
KH₂PO₄ 7g/L
NaOH 1g/L
KOH 2.5g/L
citric acid 0.5g/L
trace mineral solution 5 mL
The trace mineral solution contained:
nitrilotriacetate (70mM, pH 6.5)
ferric citrate (7.5 g/L)
MnCl₂.4H₂O (1.3 g/L)
CoCl₂.6H₂O (0.21 g/L)
CuCl₂.2H₂O (0.13 g/L)
H₃BO₃ (0.25 g/L)
ZnSO₄.7H₂O (1.2 g/L)
Na₂MoO₄.2H₂O (0.15 g/L)

### 1.2 Preparation of preculture

A sterile plastic tube containing 10 mL of LB medium, ampicillin (50mg/mL) and tetracycline (15mg/mL) was inoculated with a single colony of *E*. *coli* JM107-nanA-(Nst-O1, pBBnsy) and the preculture grown at 37 °C on a rocking table (190rpm) for 16 h.

### 1.3 Bacterial Growth

A sterile 1L triple-baffled flask containing 100 mL minimum glycerol medium, ampicillin (50mg/mL), tetracyclin (15mg/mL), thiamine (4.5 mg/L) and MgSO₄.7H₂O 1M (100 µL) was inoculated with 5 mL of *E*. *coli* JM107-nanA-(Nst-01, pBBnsy) preculture and the resulting culture grown at 37 °C for 8h. Bacterial growth was monitored by OD at 540 nm, and after 8h the OD is normally about 1.

### 1.4 Reaction

When the OD₅₄₀ is about 1, isopropyl 1-thio-β-galactopyranoside (IPTG) (20 µL of a 50mg/mL solution) and the substrates 6-*S*-Neu5Ac (5 mg, 0.0154mmol) and allyl β-lactoside (11.75mg, 0.0308 mmol, 2 equivalents) were added via a sterile filter and the culture was shaken at 28 °C for 16h. After 16h the OD₅₄₀ is normally between 4 and 10 and the culture is stopped. The cells were recovered by centrifugation at 8000g and 4 °C for 10 min. The supernatant i.e. the extracellular fraction was separated for further TLC analysis while the pellet was resuspended in 10 mL H₂O and boiled for 30 min. The resulting suspension was again centrifuged (8000g, 4 °C for 10 min). The supernatant (intracellular fraction) was mixed with 0.5g activated charcoal, filtered through Celite and washed with 20mL distilled water. The adsorbed oligosaccharides were desorbed with 60 mL 50% aq. EtOH. When mentioned the oligosaccharides were further purified on a DOWEX exchange ions resin (DOWEX 1x4 50) and silica gel column chromatography.

### 1.5 Analysis of the reaction products

TLC analysis (BuOH, AcOH, H₂O v/v 2/1/1) of the crude intracellular fraction revealed the presence of a new compound with the same mobility as allyl 3'-(sialyl)-lactoside. Its presence in the extracellular fraction is not detected at this stage because of the high concentration of inorganic salts (Figure 2)

Mass spectrometry of intracellular and extracellular fractions after purification on activated charcoal both indicated the presence of the expected trisaccharide although it is essentially localised in the intracellular fraction.

The intracellular fraction was further purified by a resin column DOWEX 1x4. The resin (8 mL) was conditioned in a 20 mL plastic syringe and successively washed with 100 mL of 1 N NaOH, 100 mL of 1 M NaHCO₃ and washed H₂O. The mixture was loaded on the resin, washed with H₂O and eluted with NaHCO₃ 50mM. The resorcinol positive fractions were collected and neutralized with Amberlite (120 IR) H⁺, filtered and lyophilised.

The purified fractions were combined and lyophilised to give 13.3mg white solid.

### This purified fraction was further purified by column chromatography on silica gel. Eluent:

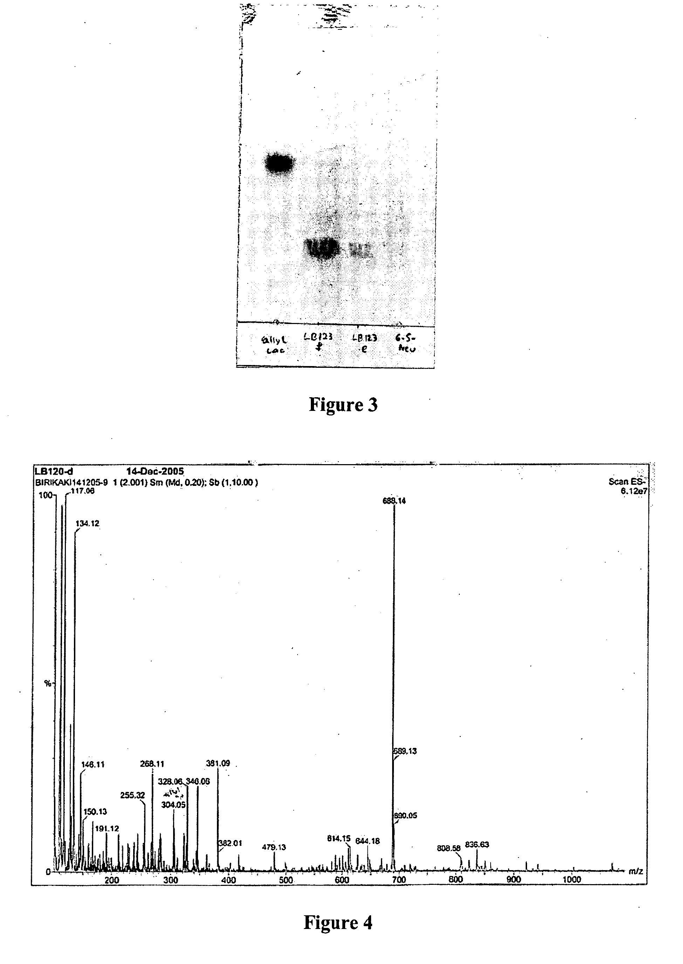

CH₃CN:H₂O 9:1. Obtained fraction (0.8 mg) - see Figure 3.

The production of the compound is indicated by the peak at m/z = 688 corresponding to [M-Na]-. The peak at 381 corresponds to fragment resulting from the loss of the (6-*S*-Neu5Ac) The isotopic distribution is in agreement with the expected formula m/z 688 (100%) 689 (31%) 690 (12%) indicated the presence of the sulfur atom in the molecule (Figure 4).

### Example 2 : In vitro synthesis of Allyl [(5-N-Acetyl-6-deoxy-6-thio-α-neuraminyl)-(2→ 3)]-β-D-gatactopyranosyl-(1→ 4)-β-D-glucopyranoside with crude cellular extract of E. coli JM107-nanA-(Nst-01, pBBnsy) strain in presence of CTP, allyl β-lactoside and 6-S-Neu5Ac.

In this aspect, the *E. coli* JM107-nanA-(Nst-01, pBBnsy) strain was grown in a shake flask fermentation and lysed before *in vitro* glycosylation reaction described in the scheme below.

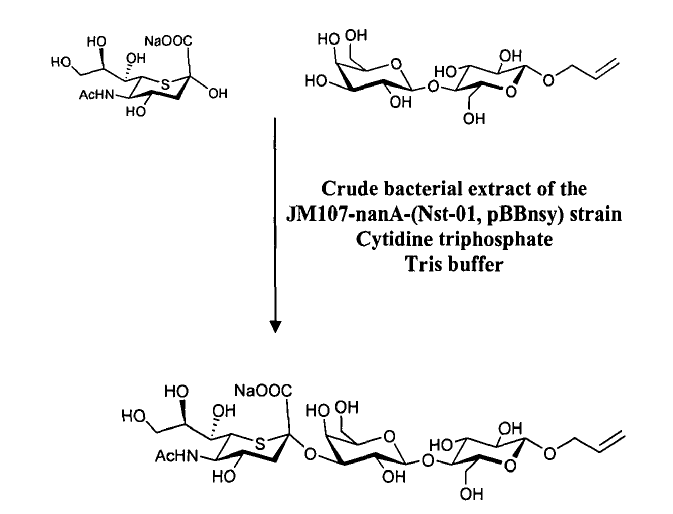

### 2.1 Growth of colonies

Agar plates consisted of LB solid medium, ampicillin (50 mg/L) and tetracyclin (15 mg/L). *E. coli* JM107-nanA-(Nst-01, pBBnsy) cells were streaked onto the plate and incubated at 37 °C for 24 h.
Only single bacterial colony was selected for preculture preparations.

### 2.2 Preparation of preculture

A sterile plastic tube containing 7.5 mL LB liquid medium, ampicillin (50 mg/L) and tetracyclin (15 mg/L) was inoculated with a single colony of *E*. *coli* JM107-nanA-(Nst-O1, pBBnsy) and the preculture grown at 37 °C on a rocking table (190rpm) for 15 h.

### 2.3 Bacterial Growth

A sterile 1L triple-baffled flask containing 100 mL LB medium, ampicillin (50 mg/L) and tetracyclin (15 mg/L) was inoculated with 3 mL *E. coli* JM107-nanA-(Nst-01, pBBnsy) preculture and the resulting culture grown at 37 °C for 4h. Cell growth was monitored by OD at 600 nm. The optical density at 600nm after 4h is usually between 0.6 and 0.8. Isopropyl 1-thio-β-galactopyranoside (IPTG) (300 µL of a 50mg/mL solution), a gratuitous inducer of protein synthesis, was added via a sterile filter and the cells grown for an additional 3h at 37 °C on a rocking table (190rpm).

### 2.4 Preparation of crude cellular extract

The culture was transferred into plastic tubes at 0 °C (ice-bath) and centrifuged at 5000 rpm and 4 °C for 7 min. The supernatant was decanted and the cell pellet resuspended in 6 mL Tris HCl (50 mM, pH 7.5) and again centrifuged as above. The cell pellet was resuspended in 3 mL Tris HCl (50 mM, pH 7.5). The cells were lysed by sonication (20 kHz) with 5 sec pulse between 10 sec intervals for 10 min. During cell disruption the U-shaped cylindrical tube containing the sample was kept in an ice-bath to prevent overheating.
The solution was centrifuged at 13000g and 4 °C for 15 min. The supernatant was aliquoted and stored at -20 °C.

### 2.5 In vitro reaction with crude cellular extract

The in vitro synthesis was performed with the crude bacterial extract of metabolically engineered *E. coli* strain expressing CMP-sialic acid synthetase and sialyltransferase prepared as described above.

The reaction contained 6-*S*-Neu5Ac (5.00 mg, 0.0154mmol), allyl β-lactoside (11.75mg, 0.0308 mmol), cytidine 5'-triphosphate disodium salt (CTP, 8.1mg, 0.0154mmol), 4-(2-hydroxyethyl)piperazine-1-ethanesulfonic acid (HEPES, 63mg, 0.26mmol), MgCl₂.6H₂O (22mg, 0.108mmo1), 1,4-dithioerythritol (DET, 1mg, 6.5x10⁻³mmol), inorganic pyrophosphatase (300 mU) in 2 mL of the crude cellular extract and performed at 30 °C for 24h. The reaction was followed by TLC analysis (BuOH, AcOH, H₂O v/v 2/1/1). After completion the mixture was centrifuged at 13000g and 4 °C for 15 min. The pellet was resuspended in 2 mL H₂O and centrifuged to recover all oligosaccharides and this was repeated three times. The combined supernatant fractions were purified on a Sephadex G10 column and eluted with H₂O to afford a main product with identical spectral properties to those reported for compound isolated from the *in vivo* synthesis (example 1).

### REFERENCES

1. Varki, A., Diversity in the sialic acids. Glycobiology 1992, 2, (1), 25-40.
2. Varki, A., Sialic acids as ligands in recognition phenomena. FASEB Journal 1997, 11, (4), 248-255.
3. Angata, T.; Varki, A., Chemical Diversity in the Sialic Acids and Related α-Keto Acids: An Evolutionary Perspective. Chemical Reviews 2002, 102, (2), 439-469.
4. Dwek, R. A., Glycobiology: Toward Understanding the Function of Sugars. Chem. Rev. 1996, 96, (2), 683-720.
5. Stockert, R. J., The asialoglycoprotein receptor: relationships between structure, function, and expression. Physiological Reviews 1995, 75, (3), 591-609.
6. Maynard, Y.; Baenziger, J. U., Oligosaccharide specific endocytosis by isolated rat hepatic reticuloendothelial cells. The Journal of Biological Chemistry 1981, 256, (15), 8063-8.
7. Elliott, S.; Lorenzini, T.; Asher, S.; Aoki, K.; Brankow, D.; Buck, L.; Busse, L.; Chang, D.; Fuller, J.; Grant, J.; Hernday, N.; Hokum, M.; Hu, S.; Knudten, A.; Levin, N.; Komorowski, R.; Martin, F.; Navarro, R.; Osslund, T.; Rogers, G.; Rogers, N.; Trail, G.; Egrie, J., Enhancement of therapeutic protein in vivo activities through glycoengineering. Nature Biotechnology 2003, 21, (4), 414-421.
8. Cumming, D. A., Glycosylation of recombinant protein therapeutics: control and functional implications. Glycobiology 1991, 1, (2), 115-30.
9. Sethuraman, N.; Stadheim, T. A., Challenges in therapeutic glycoprotein production. Current Opinion in Biotechnology 2006, 17, (4), 341-346.
10. Taylor, G., Sialidases: structures, biological significance and therapeutic potential. Current Opinion in Structural Biology 1996, 6, (6), 830-837.
11. Roggentin, P.; Schauer, R.; Hoyer, L. L.; Vimr, E. R., The sialidase superfamily and its spread by horizontal gene transfer. Molecular microbiology 1993, 9, (5), 915-21.
12. Colman, P. M., Influenza virus neuraminidase: structure, antibodies, and inhibitors. Protein Science 1994, 3, (10), 1687-96.
13. Corfield, T., Bacterial sialidases. Roles in pathogenicity and nutrition. Glycobiology 1992, 2, (6), 509-21.
14. Stamatos, N. M.; Liang, F.; Nan, X.; Landry, K.; Cross, A. S.; Wang, L.-X.; Pshezhetsky, A. V., Differential expression of endogenous sialidases of human monocytes during cellular differentiation into macrophages. FEBS Journal 2005, 272, (10), 2545-2556.
15. Achyuthan, K. E.; Achyuthan, A. M., Comparative enzymology, biochemistry and pathophysiology of human exo-α-sialidases (neuraminidases). Comparative Biochemistry and Physiology, Part B: Biochemistry & Molecular Biology 2001, 129B, (1), 29-64.
16. Mueller, O. T.; Henry, W. M.; Haley, L. L.; Byers, M. G.; Eddy, R. L.; Shows, T. B., Sialidosis and galactosialidosis: chromosomal assignment of two genes associated with neuraminidase-deficiency disorders. Proceedings of the National Academy of Sciences of the United States of America 1986, 83, (6), 1817-21.
17. Miyagi, T.; Kato, K.; Ueno, S.; Wada, T., Aberrant expression of sialidase in cancer. Trends in Glycoscience and Glycotechnology 2004, 16, (92), 371-381.
18. Driguez, H., Thiooligosaccharides as Tools for Structural Biology. ChemBioChem 2001, 2, (5), 311-318.
19. Bundle, D. R.; Nitz, M.; Cutler, J. E. Synthetic anti-Candida albicans oligosaccharide-based vaccines. US2006058506.
20. Rich, J. R.; Wakarchuk, W. W.; Bundle, D. R., Chemical and Chemoenzymatic Synthesis of S-Linked Ganglioside Analogues and Their Protein Conjugates for Use as Immunogens. Chemistry - A European Journal 2006, 12, (3), 845-858.
21. Bundle, D. R.; Rich, J. R.; Jacques, S.; Yu, H. N.; Nitz, M.; Ling, C.-C., Thiooligosaccharide conjugate vaccines evoke antibodies specific for native antigens. Angewandte Chemie, International Edition 2005, 44, (47), 7725-7729.
22. Zhang, J.; Wu, B.; Liu, Z.; Kowal, P.; Chen, X.; Shao, J.; Wang, P. G., Large-scale synthesis of carbohydrates for pharmaceutical development. Current Organic Chemistry 2001, 5, (12), 1169-1176.
23. Jahn, M.; Withers, S. G., New Approaches to Enzymatic Oligosaccharide Synthesis: Glycosynthases and Thioglycoligases. Biocatalysis and Biotransformation 2003, 21, (4/5), 159-166.
24. Jahn, M.; Marles, J.; Warren, R. A. J.; Withers, S. G., Thioglycoligase: mutant glycosidases for thioglycoside synthesis. Angewandte Chemie, International Edition 2003, 42, (3), 352-354.
25. Mullegger, J.; Chen, H.-M.; Chan, W. Y.; Reid, S. P.; Jahn, M.; Antony, R.; Warren, J.; Salleh, H. M.; Withers, S. G., Thermostable glycosynthases and thioglycoligases derived from Thermotoga maritima β-glucuronidase. ChemBioChem 2006, 7, (7), 1028-1030.
26. Mullegger, J.; Chen, H. M.; Warren, R. A. J.; Withers, S. G., Glycosylation of a neoglycoprotein by using glycosynthase and thioglycoligase approaches: the generation of a thioglycoprotein. Angewandte Chemie, International Edition 2006, 45, (16), 2585-2588.
27. Rich, J. R.; Szpacenko, A.; Palcic, M. M.; Bundle, D. R., Glycosyltransferase-catalyzed synthesis of thiooligosaccharides. Angewandte Chemie, International Edition 2004, 43, (5), 613-615.
28. Aharoni, A.; Thieme, K.; Chiu, C. P. C.; Buchini, S.; Lairson, L. L.; Chen, H.; Strynadka, N. C. J.; Wakarchuk, W. W.; Withers, S. G., High-throughput screening methodology for the directed evolution of glycosyltransferases. Nature Methods 2006, 3, (8), 609-614.
29. Robina, I.; Vogel, P., The synthesis of disaccharides, oligosaccharides and analogues containing thiosugars. Current Organic Chemistry 2002, 6, (5), 471-491.
30. Shin, J. E. N.; Maradufu, A.; Marion, J.; Perlin, A. S., Specificity of α- and β-D-galactosidase towards analogs of D-galactopyranosides modified at C-4 or C-5. Carbohydrate Research 1980, 84, (2), 328-35.
31. Yuasa, H.; Matsuura, S.; Hashimoto, H., Synthesis of 5-thiomannose-containing oligomannoside mimics: binding abilities to concanavalin A. Bioorganic & Medicinal Chemistry Letters 1998, 8, (11), 1297-1300.
32. Tsuruta, O.; Yuasa, H.; Kurono, S.; Hashimoto, H., Syntheses of two trimannose analogs each containing C-mannosyl or 5-thio-C-mannosyl residue: their affinities to concanavalin A. Bioorganic & Medicinal Chemistry Letters 1999, 9, (6), 807-810.
33. Tsuruta, O.; Yuasa, H.; Hashimoto, H.; Kurono, S.; Yazawa, S., Affinity of 5-thio-L-fucose-containing Lewis X (Lex) trisaccharide analogs to anti-Lex monoclonal antibody. Bioorganic & Medicinal Chemistry Letters 1999, 9, (7), 1019-1022.
34. Bellamy, F.; Barberousse, V.; Martin, N.; Masson, P.; Millet, J.; Samreth, S.; Sepulchre, C.; Theveniaux, J.; Horton, D., Thioxyloside derivatives as orally active venous antithrombotics. European Journal of Medicinal Chemistry 1995, 30.
35. Barberousse, V.; Samreth, S.; Boubia, B.; Bellamy, F.; Peyrou, V. Preparation of thio-xylose glycosides as antithrombotic agents. EP1668021.
36. Yuasa, H.; Hindsgaul, O.; Palcic, M. M., Chemical-enzymic synthesis of 5'-thio-N-acetyllactosamine: the first disaccharide with sulfur in the ring of the non-reducing sugar. Journal of the American Chemical Society 1992, 114, (14), 5891-2.
37. Mack, H.; Brossmer, R., Synthesis of 6-thiosialic acids and 6-thio-N-acetyl-D-neuraminic acid. Tetrahedron Letters 1987, 28, (2), 191-4.
38. Mack, H.; Brossmer, R., Synthesis of 6-thio-sialic acids. Tetrahedron 1998, 54, (18), 4521-4538.
39. Kok, G. B.; Campbell, M.; Mickey, B.; von Itzstein, M., Synthesis and biological evaluation of sulfur isosteres of the potent influenza virus sialidase inhibitors 4-amino-4-deoxy- and 4-deoxy-4-guanidino-Neu5Ac2en. Journal of the Chemical Society, Perkin Transactions 1: Organic and Bio-Organic Chemistry 1996, (23), 2811-2815.
40. Tsuruta, O.; Yuasa, H.; Hashimoto, H.; Sujino, K.; Otter, A.; Li, H.; Palcic, M. M., Synthesis of GDP-5-thiosugars and Their Use as Glycosyl Donor Substrates for Glycosyltransferases. Journal of Organic Chemistry 2003, 68, (16), 6400-6406.
41. Goon, S.; Bertozzi, C. R., Metabolic substrate engineering as a tool for glycobiology. Journal of Carbohydrate Chemistry 2002, 21, (7-9), 943-977.
42. Yu, H.; Chokhawala, H.; Karpel, R.; Yu, H.; Wu, B.; Zhang, J.; Zhang, Y.; Jia, Q.; Chen, X., A Multifunctional Pasteurella multocida Sialyltransferase: A Powerful Tool for the Synthesis of Sialoside Libraries. Journal of the American Chemical Society 2005, 127, (50), 17618-17619.
43. Zou, W.; Borrelli, S.; Gilbert, M.; Liu, T.; Pon, R. A.; Jennings, H. J., Bioengineering of Surface GD3 Ganglioside for Immunotargeting Human Melanoma Cells. Journal of Biological Chemistry 2004, 279, (24), 25390-25399.
44. Pan, Y.; Chefalo, P.; Nagy, N.; Harding, C.; Guo, Z., Synthesis and Immunological Properties of N-Modified GM3 Antigens as Therapeutic Cancer Vaccines. Journal of Medicinal Chemistry 2005, 48, (3), 875-883.
45. Keppler, O. T.; Herrmann, M.; Von Der Lieth, C. W.; Stehling, P.; Reutter, W.; Pawlita, M., Elongation of the N-acyl side chain of sialic acids in MDCK II cells inhibits influenza A virus infection. Biochemical and Biophysical Research Communications 1998, 253, (2), 437-442.
46. Buttner, B.; Kannicht, C.; Schmidt, C.; Loster, K.; Reutter, W.; Lee, H.-Y.; Nohring, S.; Horstkorte, R., Biochemical engineering of cell surface sialic acids stimulates axonal growth. Journal of Neuroscience 2002, 22, (20), 8869-8875.
47. Tullius, M. V.; Munson, R. S., Jr.; Wang, J.; Gibson, B. W., Purification, Cloning, and Expression of a Cytidine 5'-Monophosphate N-Acetylneuraminic Acid Synthetase from Haemophilus ducreyi. J. Biol. Chem. 1996, 271, (26), 15373-15380.
48. Vionnet, J.; Concepcion, N.; Warner, T.; Zapata, G.; Hanover, J.; Vann, W. F., Purification of CMP-N-acetylneuraminic acid synthetase from bovine anterior pituitary glands. Glycobiology 1999, 9, (5), 481-487.
49. Gilbert, M.; Watson, D. C.; Wakarchuk, W. W., Purification and characterization of the recombinant CMP-sialic acid synthetase from Neisseria meningitidis. Biotechnology Letters 1997, 19, (5), 417-420.
50. Priem, B.; Gilbert, M.; Wakarchuk, W. W.; Heyraud, A.; Samain, E., A new fermentation process allows large-scale production of human milk oligosaccharides by metabolically engineered bacteria. Glycobiology 2002, 12, (4), 235-240.
51. Karwaski, M.-F.; Wakarchuk, W. W.; Gilbert, M., High-level expression of recombinant Neisseria CMP-sialic acid synthetase in Escherichia coli. Protein Expression and Purification 2002, 25, (2), 237-240.
52. Harduin-Lepers, A.; Recchi, M.-A.; Delannoy, P., 1994, the year of sialyltransferases. Glycobiology 1995, 5, (8), 741-758.
53. Harduin-Lepers, A.; Vallejo-Ruiz, V.; Krzewinski-Recchi, M. A.; Samyn-Petit, B.; Julien, S.; Delannoy, P., The human sialyltransferase family. Biochimie 2001, 83, (8), 727-737.
54. Izumi, M.; Wong, C.-H., Microbial sialyltransferases for carbohydrate synthesis. Trends in Glycoscience and Glycotechnology 2001, 13, (72), 345-360.
55. Harduin-Lepers, A.; Mollicone, R.; Delannoy, P.; Oriol, R., The animal sialyltransferases and sialyltransferase-related genes: a phylogenetic approach. Glycobiology 2005, 15, (8), 805-817.
56. Tsuji, S.; Datta, A. K.; Paulson, J. C., Systematic nomenclature for sialyltransferases. Glycobiology 1996, 6, (7), v-vii.
57. Lau, J. T. Y.; Wuensch, S. A., Sialyltransferases. Carbohydrates in Chemistry and Biology, Vol. 3, (Eds: B. Ernst, G. W. Hart, P. Sinay), Wiley-VCH, Weinheim, 2000, 3, 213-226.
58. Inoue, Y.; Inoue, S., Diversity in sialic and polysialic acid residues and related enzymes. Pure and Applied Chemistry 1999, 71, (5), 789-800.
59. Rutishauser, U.; Acheson, A.; Hall, A. K.; Mann, D. M.; Sunshine, J., The neural cell adhesion molecule (NCAM) as a regulator of cell-cell interactions. Science 1988, 240, (4848), 53-57.
60. Chotigeat, W.; Chayanunnukul, W.; Phongdara, A., Expression of a mammalian a2,6-sialyltransferase gene in Pichia pastoris. Journal of Biotechnology 2000, 81, (1), 55-61.
61. Sievi, E.; Helin, J.; Heikinheimo, R.; Makarow, M., Glycan engineering of proteins with whole living yeast cells expressing rat liver α2,3-sialyltransferase in the porous cell wall. FEBS Letters 1998, 441, (2), 177-180.
62. Hamilton, S. R.; Davidson, R. C.; Sethuraman, N.; Nett, J. H.; Jiang, Y.; Rios, S.; Bobrowicz, P.; Stadheim, T. A.; Li, H.; Choi, B.-K.; Hopkins, D.; Wischnewski, H.; Roser, J.; Mitchell, T.; Strawbridge, R. R.; Hoopes, J.; Wildt, S.; Gerngross, T. U., Humanization of Yeast to Produce Complex Terminally Sialylated Glycoproteins. Science 2006, 313, (5792), 1441-1443.
63. DeFrees, S.; Wang, Z.-G.; Xing, R.; Scott, A. E.; Wang, J.; Zopf, D.; Gouty, D. L.; Sjoberg, E. R.; Panneerselvam, K.; Brinkman-Van der Linden, E. C. M.; Bayer, R. J.; Tarp, M. A.; Clausen, H., GlycoPEGylation of recombinant therapeutic proteins produced in Escherichia coli. Glycobiology 2006, 16, (9), 833-843.
64. Langenhan, J. M.; Thorson, J. S., Recent carbohydrate-based chemoselective ligation applications. Current Organic Synthesis 2005, 2, (1), 59-81.
65. Fort, S.; Birikaki, L.; Dubois, M.-P.; Antoine, T.; Samain, E.; Driguez, H., Biosynthesis of conjugatable saccharidic moieties of GM2 and GM3 gangliosides by engineered E. coli. Chemical Communications 2005, (20), 2558-2560.

## Claims

1. A method of preparing 6-thio-sialyl-glycosyl compounds, comprising the step consisting of reacting *in vitro* or *in vivo* 6-thio-sialic acid compounds selected from 6-thio-sialic acid, *N-* or *O*-acyl derivative thereof or a derivative of formula I: 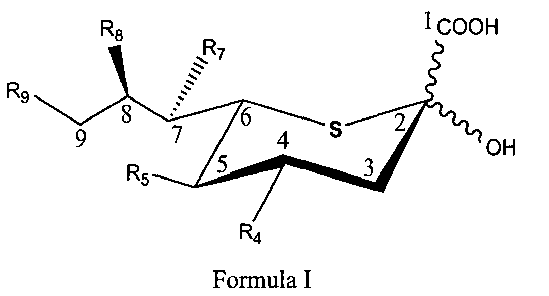 Wherein
R4 is OH, *O*-acetyl, *O*-galactosyl, *O*-fucosyl, or N₃
R5 is NH₂, OH, N₃, NHCOR' (R'= CH₃, CH₂Ph, CH₂N₃, CH=CHCH₃, CH₂CH₂COCH₃, (CH₂)ₙCH₃ where n is ≥1, or L-OCH₂CH₂(OCH₂CH₂)ₙOCH₃) wherein L is a substituted or unsubstituted alkyl or heteroalkylgroup.
R7 is OH, *O*-acetyl, or NHCOR' ; R' being defined as above
R8 is OH, *O*-acetyl, *O*-methyl, sulfate, *O*-sialyl, or O-glucosyl
and R9 is OH, *O*-acetyl, *O*-lactyl, phosphate, *O*-sialyl, F, or N_{3;}
with a CMP-sialic synthetase to form CMP-6-thio sialic acid compounds and with a sialyltransferase to transfer said CMP-6-thio sialic acid compounds as donor substrate to glycosyl compounds as acceptor subtrate leading to the enzymatic coupling of 6-thiosialic acid compounds onto glycosyl compounds.

2. The method according to claim 1, wherein glycosyl compounds are selected from glycoproteins, glycolipids and oligosaccharides.

3. The method according to claim 1 to produce glycoproteins harbouring 6-thio-sialic acid, *N-*or *O*-acyl derivative thereof or a derivative of formula I.

4. The method according to claim 1 to produce humanized glycoproteins wherein sialic acid residue(s) is replaced by 6-thio-sialic acid, *N-* or *O*-acyl derivative thereof or a derivative of formula I.

5. The method according to claim 1, wherein the enzymatic coupling of 6-thiosialic acid compounds onto glycosyl compounds is performed using a metabolically engineered microorganism expressing a CMP-sialic synthetase and a sialyltransferase.

6. The method according to claim 1, wherein the enzymatic coupling of 6-thiosialic acid compounds onto glycosyl compounds is performed by *in vitro* using a crude enzymatic extract.

7. The method according to claim 1, wherein the enzymatic coupling of 6-thiosialic acid compounds onto glycosyl compounds is performed by *in vitro* using purified CMP-sialic synthetase and sialyltransferase.

8. The method according to claim 1, wherein 6-thio sialic acid compounds are 6-thio-NeuAc (R_{4,7,8,9} = OH, R₅ = NHAc), 6-Thio-*N*-glycolylneuraminic acid (R_{4,5,7,8,9} = OH) or 6-thio-*N-*acyl-neuraminic acid (R_{4,7,8,9} = OH, R₅= NHCOR' where R' is a substituted or unsubstituted alkyl or heteroalkyl chain).

9. The method according to claim 1, wherein said CMP sialic acid synthase is from *Neisseria meningitidis,* such as the CMP sialic acid synthase encoded by SEQ ID No 6.

10. The method according to claim 1, wherein said CMP sialic acid synthase is encoded by a *neuA* gene, such as the *neuA* from C. jejuni strain ATCC accession number 43438.

11. The method according to claim 1, wherein said sialyltransferase is selected from ST3, ST6, and ST8 families that transfers sialic acids to the 3-OH, 6-OH or 8-OH hydroxyl positions of the underlying carbohydrate respectively.

12. The method according to claim 11, wherein said sialyl transferase is from mammals, bacteria, fish, birds or amphibians.

13. The method according to claim 11, wherein depending on the acceptor, one sialyltransferase as listed below is selected:
| Name | Other name | Preferred acceptor^{a} |
|---|---|---|
| **2,3-STs** | | |
| ST3Gal I | ST-30, ST3GalA.1, ST-2 | **Gal**β1,3GalNAc |
| ST3Gal II | ST3GalA.2, SAT-IV | **Gal**β1,3GalNAc |
| ST3Gal III | ST-3N, ST-3 | **Gal**β1,3GlcNAc |
| ST3Gal IV | ST-Z, SAT-3, ST-4 | **Gal**β1,4GlcNAc |
| | | |
| **2,6-STs** | | |
|---|---|---|
| ST6Gal I | ST-6N, SiaT-1, ST-1 | **Gal**β1,4GlcNAc |
| ST6GalNAc I | ST3O-I | **GalNAc**aαSer/Thr |
| | | Galβ1,3**GalNAc**αSer/Thr |
| | | Siaα2-3Galβ1,3**GalNAc**αSer/Thr |
| ST6GalNAc II | | Siaα2-3Galβ1,3**GalNAc**αSer/Thr |
| | | Galβ1,3**GalNAc**aSer/Thr |
| ST6GlcNAc III | STY, ST60-II | Siaα2-3Galβ1,3**GalNAc**αSer/Thr |
| ST6GIcNAc | | (Siaα2-3Galβ1-3)**GlcNAc**βR |
| | | |
| **2,8-STs** | | |
|---|---|---|
| ST8Sia I | SAT-II (SAT-III), GD3 synthase | GM3 (a-series) |
| | | |
| ST8Sia II | STX | glycoprotein |
| ST8Sia III | | Siaα2-3Galβ1,4GlcNAc |
| | | GD3 (b-series) |
| ST8Sia IV | PST-1 | glycoprotein |
| STBSia V | SAT-V/-SAT-III | GD1a, GT1b, GD3 |

14. The method according to claim 11, wherein said sialyltransferase is a α-2,3-sialytransferase from *Neisseria meningitidis,* such as SEQ ID No 4.

15. The method according to claim 14, to transfer 6-thio acid sialic compounds onto galactosyl acceptors.

16. The method according to claim 1, for the sialylation with 6-thio-sialic acid of glycoproteins harbouring glycans terminated by a Gal, GalNAc, GlcNac or Sia residue, such as glycoproteins comprising glycans comprising biantennary GlcNac2Man3Gal(0, 1, or 2) structures with or without bisecting GlcNac.

17. The method according to claim 1 or 16, wherein glycoproteins are produced by expression systems such as mammalian cells (i.e Chinese hamster ovary cells CHO), insect cells, yeasts (*P. pastoris, Saccharomyces cerevisae*) or bacteria (*E. coli*).

18. The method according to claim 1 for transfering a 6-thio sialic acid compound onto glycoproteins obtained in genetically engineered yeasts which comprises alpha 1,6-mannosyl-transferase inactivation and expression of at least one of heterologous mannosidases, glycosyltransferases, galactosyltransferases and/or sialyltransferases.

19. The method according to claim 18, wherein said glycoproteins are incubated *in vitro* with a CMP-sialic synthetase and a sialyltransferase to introduce said 6-thio-sialic acid compound.

20. A method for producing 6-thio sialylated glycoproteins in genetically engineered yeasts which comprises alpha 1,6-mannosyl-transferase inactivation and expression of at least one of heterologous mannosidases, glycosyltransferases, galactosyltransferases and/or sialyltransferases, said yeast being further modified to express an heterologous gene encoding a sialyl transporter, such as for example a *nanT* gene; said yeasts being cultured in a culture medium comprising said 6-thio-sialic acid compounds.

21. A method for producing 6-thio sialylated glycoproteins in genetically modified mammalian cells, such as CHO, which comprises the expression of at least one of heterologous glycosyltransferase, such as a galactosyltransferase, and a sialyltransferase, said mammalian cells being further modified to express an heterologous gene encoding a sialyl transporter, such as for example a *nanT* gene; and being cultured in a culture medium comprising said 6-thio-sialic acid compounds.

22. The method according to one of claims 1 to 18, wherein the glycosyl acceptor is a mono, oligo or polysaccharide whose reducing end is functionalized by a chemical group allowing subsequent chemical or enzymatic conjugation to another chemical species such as a solid support, a peptide, a protein, or an oligonucleotide, and wherein said glycosyl acceptor is defined by the following formula II :

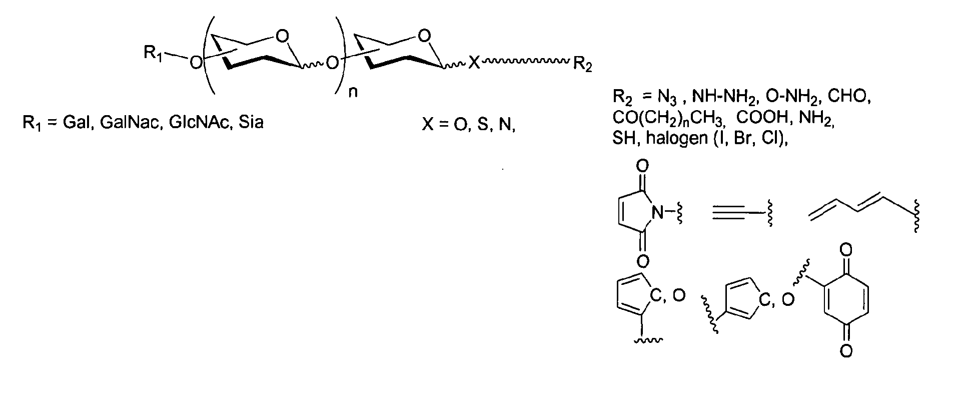

23. The method according to claim 22, wherein said conjugation reaction includes a cycloaddition reaction such as 1,3-cyclodipolar addition ("Click chemistry") between azide and alkyne groups catalyzed by Cu (I), or Diels Alder reactions.

24. The method according to claim 22, wherein said conjugation reaction includes a nucleophilic addition reaction between amine, hydroxylamine, hydrazine and aldehyde or activated carboxylic acid as well as between thiol and maleimide or halogen.

25. The method according to claim 21, wherein the glycosyl acceptor is allyl β-lactoside (X = O, R2 = CH₂CH=CH₂).

26. The method according to one of claims 21 to 25, wherein a recombinant protein is first reacted with said glycosyl acceptor and then is incubated with a sialyltransferase to introduce said 6-thio-sialic acid compounds.

27. The method according to claim 21 to 25, wherein said recombinant protein reacted with said glycosyl acceptor is incubated with a cell extract comprising a CMP-sialic synthetase and a sialyltransferase or with a purified CMP-sialic synthetase and sialyltransferase.

28. The method according to claim 1, which is performed *in vivo,* comprising the step consisting of culturing a microorganism in a culture medium comprising said 6-thio-sialic acid compounds, wherein said microorganism comprises heterologous genes encoding a CMP-Neu5Ac synthetase, and a sialyltransferase, said microorganism expressing a sialic acid transporter, such as NanT, and a beta-galactoside permease (LacY); and wherein the endogenous genes coding for sialic acid aldolase (*NanA*) and optionally for ManNac kinase (*NanK*) have been deleted or inactivated.

29. The method according to claim 28, wherein said microorganism is LacZ-.

30. The method according to claim 28, wherein said microorganism is grown on glycerol as carbon source.

31. The method according to claim 28, wherein said micoorganism further expresses a recombinant protein or peptide, or a recombinant antibody.

32. The method according to claim 28, wherein the heterologous sialyltransferase gene is selected from α-2,3-sialyltransferase, α-2,3- and α-2,8-sialyltransferase (*cstII*), and α-2,6 sialyltransferase.

33. The method according to claim 28, wherein the heterologous CMP-Neu5Ac synthetase is a *neuA* gene isolated from bacterial strains that contains sialylated structure in their cells envelope, such as *C. jejuni* strain ATCC Accession No. 43438.

34. The method according to one of claims 28 to 33, wherein said microorganism is a E. coli strain which is *LacY*+ (β-galactoside permease), *LacZ*- (β galactosidase), and optionally *MelA*- (α-galactosidase).

35. The method according to one of claims 1 to 7, wherein the glycosyl acceptor is synthesized internally starting from an exogenous precursor added in the culture medium selected from lactose, galactose, β-galactoside, and α-galactoside.

36. A microorganism as defined in one of claims 20 and 28 to 33.

37. A cell as defined in claim 21.

38. A cell culture medium comprising a 6-thio-sialic acid compound as defined in claim 1 and the microorganism of claim 36 or the cell of claim 37.

39. A 6-thio-sialylated recombinant glycoprotein or antibody obtainable by the method according to one of claims 1 to 35.

40. A pharmaceutical composition comprising as active ingredient a 6-thio-sialylated recombinant glycoprotein or antibody obtainable by the method according to one of claims 1 to 35.

41. The use of a sialyltransferase to transfer a 6-thio-sialic acid compound as defined in claim 1 onto a glycosyl acceptor as defined in claim 22.
